# EUROPEAN PATENT SPECIFICATION

(11) **EP 3 001 809 B1**
(45) Date of publication and mention of the grant of the patent: **12.07.2017**
(21) Application number: 14758005.4
(22) Date of filing: 01.08.2014
(51) Int. Cl.: A61K 8/362, A61K 8/41, A61Q 5/00, A61Q 5/04, A61Q 5/08, A61Q 5/10

(54) **METHOD FOR CONDITIONING HAIR AND SKIN**
VERFAHREN ZUM KONDITIONIEREN VON HAAR UND HAUT
PROCÉDÉS DE CONDITIONNEMENT CAPILLAIRE ET CUTANÉ

(30) Priority: 01.08.2013 US 201361861281 P; 20.08.2013 US 201361867872 P; 02.10.2013 US 201361885898 P; 12.11.2013 US 201361903239 P; 21.04.2014 US 201414257089; 21.04.2014 US 201414257056; 21.04.2014 US 201414257076; 19.05.2014 US 201462000340 P
(43) Date of publication of application: 06.04.2016
(60) Divisional application: 17163334.0
(73) Proprietor: LIQWD, INC., Santa Barbara, California 93108 (US)
(72) Inventor: PRESSLY, Eric D., Santa Barbara, California 93108 (US); HAWKER, Craig J., Santa Barbara, California 93108 (US)
(74) Representative: Potter Clarkson LLP
(86) International application number: PCT/US2014/049388
(87) International publication number: WO 2015/017768

(56) References cited:
- EP-A2- 1 174 112
- WO-A1-2014/125452
- DE-A1- 10 051 773
- DE-A1- 10 051 774
- DE-A1-102004 052 480
- GB-A- 741 307
- GB-A- 773 559
- JP-A- 2006 327 994
- JP-A- 2009 007 283
- US-A- 3 142 623
- US-A- 3 472 243
- US-A- 4 532 950
- US-A- 5 833 966
- US-A1- 2004 086 475
- US-A1- 2006 024 257

## Description

### FIELD OF THE INVENTION

The present invention generally relates to compositions and methods for treating hair particularly for repairing disulfide bonds in hair. Compositions and methods for treating skin are also described.

### BACKGROUND OF THE INVENTION

Hair consists of many long protein chains composed of amino acid building blocks. These chains, or polymers, are bound to each other via 1) hydrogen bonding, 2) salt bridges between acid and base groups, and 3) disulfide bonds. Water reversibly cleaves the hydrogen bonds. This makes wet hair easy to shape and set. When the water evaporates, hydrogen bonds form at new positions, holding the hair in this set. In strongly acidic solutions, such as where the pH is 1.0 to 2.0, both hydrogen bonds and salt bridges are broken. The disulfide bonds, however, can still hold the protein chains together in the strand of hair under such conditions.

At a slightly alkaline pH of 8.5, some disulfide bonds are broken (Dombrink et al, Chem Matters, 1983, page 8). Repeated washing with slightly alkaline shampoo damages the hair by breaking more and more of the disulfide bonds. This causes the cuticle or outer surface of the hair strands to become ruffled and generally leaves the hair in a wet, tangled, and generally unmanageable state. This is one cause of "split ends." Once the hair dries, it is often left in a dry, rough, or frizzy condition. Additionally, rough hair catches light unevenly and makes the hair look lusterless and dull. The hair can also be left with increased levels of static upon drying, which can interfere with combing and result in a condition commonly referred to as "fly-away hair."

Disulfide linkages are also ruptured due to heating or use of various reducing treatments. Current compositions and methods for waving and straightening mammalian hair use reducing agents such as thioglycolic acid, particularly as the ammonium salt, to cleave the hair's cystine disulfide bonds. Once the disulfide bonds are broken, and the hair is placed in stress to establish the final style (e.g., straight, wavy, or curly) the disulfide bonds are reestablished. Oxidation to restore the reduced bonds can be achieved by simply exposing the hair to atmospheric oxygen, but this oxidation step is very slow and is of very little practical use. Generally, hydrogen peroxide or sodium bromate is used as the oxidizing agent. However, the newly formed disulfide bonds are under stress to maintain the hair's new shape, thus, they break easily resulting in a reversion of the hair style over time. In addition, the use of peroxides in the hair styling process can result in damaged hair, removal of non-natural color from the hair, and/or leave the hair frizzy. Furthermore, some latent free thiols may remain in the hair even after oxidative treatment.

Treatment with peroxides used in the hair styling process results in the following reaction:

2 K-S-H + H₂O₂ → K-S-S-K + 2 H₂O (Rxn I)

where K represents keratin in the hair. However, if two K-S-H groups are not present for the reaction (Rxn I) to take place, it is believed that the following reaction takes place, which results in damaged hair.

K-S-H + 3 H₂O₂ → K-SO₂-OH + 3 H₂O (Rxn II)

Keratin is also a major component in skin. Damage to the disulfide bridges of keratin can cause skin to look unhealthy or flaky. Maintaining the disulfide bridges of keratin keeps the skin healthy and prevents cracking and splitting.

A variety of approaches have been developed to alleviate these problems, including post-shampoo application of hair conditioners, such as leave-on and rinse-off products. Typically, conditioning rinses put back the oily coating, especially to the damaged portion of the hair where the cuticle has become ruffled since conditioners cling best to these portions. However, too much or too heavy a conditioner will make the hair stickier, thus attracting dirt and often may make more shampooing treatments necessary. Typically conditioners do not bind the free thiols in hair.

The use of cationic polymers to form coacervates to provide conditioning benefits to the hair is known, such as described in International Published Applications WO 93/08787 to King et al. and WO 95/01152 to Napolione et al. Commonly used cationic deposition polymers include natural polymers, such as guar gum polymers, that have been modified with cationic substituents. The selection of a cationic guar polymer with sufficient charge density and molecular weight results in sufficient deposition of conditioning agents when incorporated in a shampoo or body wash. However, a relatively high level of such cationic guar polymer generally must be deposited on the hair or skin. Moreover, the cost of such cationic guar polymer is relatively high. As a result, incorporation of cationic guar polymer can increase the manufacturing costs of such shampoo compositions. Additionally, these shampoo compositions typically are useful for wet hair conditioning, but are not capable of delivering satisfactory dry hair smooth feel. Furthermore, these conditioners do not bind the free thiols in hair.

U.S. Patent No. 5,656,265 to Bailey et al., discloses a hair styling conditioning process for use after treating the hair with a reducing agent. The process involves contacting the hair with a compound having an electrophilic group and at least one hydrophobic group. According to Bailey, the electrophilic groups react with the thiol groups to provide a plurality of hydrophobic groups on the hair. However, these conditioners do not bind the free thiols in hair together.

There is a need for hair formulations and treatments that can provide improved conditioning benefit for hair. Specifically, there is a need to provide long lasting moisturized feel, smooth feel, and manageability control to hair when it is dried. There is also a need for hair formulations and treatments that repair latent free thiols in the hair.

There is a need for hair formulations and treatments that repair and/or strengthen damaged hair and rebuild stronger bonds in hair treated with reducing agents.

There is also a need for skin formulations and treatments that provide improved conditioning and/or moisturizing benefit to the skin. In particular, there is a need to provide a long lasting moisturized and smooth feel to the skin. There is also a need for skin formulations and treatments that repair free thiols in the skin.

Therefore, it is an object of this invention to provide improved compositions and methods for repairing and/or strengthening damaged hair.

It is also an object of this invention to provide compositions and methods for using these compositions that repair and/or strengthen hair after a washing or reducing treatment.

It is also an object of this invention to provide compositions and methods for conditioning, moisturizing, and/or otherwise treating the skin.

### SUMMARY OF THE INVENTION

Compositions, kits, and methods for repairing bonds, for example, disulfide bonds, in hair or on the skin that have been damaged are disclosed. The compositions provide improved conditioning benefit for dry hair or moisturize the skin. Specifically, the compositions provide long lasting moisturized feel and smooth feel without leaving the hair greasy, improved appearance (e.g., sheen), increased dry strength (tensile strength), ease of combing the hair when wet or dried, less hair breakage, and decreased frizz. The compositions also provide a long lasting moisturized feel and smooth feel to the skin.

The compositions contain one or more compounds that interact with keratin through more than one binding events (e.g., absorption, binding, etc.) which may involve reaction with one or more thiols in the hair or on the skin. Binding herein is defined as the formation of covalent, ionic or hydrogen bonding, etc. Under normal hair washing conditions, including shampooing and conditioning, the covalent bonds formed are not succeptable to reduction or hydrolysis. Use of the binding compositions prevents reversion of the hair's repaired bonds to their free thiol state, for at least one week, two weeks, three weeks, four weeks, one month, or two months, or longer, after application of the composition.

Improved methods of styling hair, for example permanent hair waving, hair curling, and hair straightening are also provided. The binding compositions can be applied each time the hair is washed or daily, once-weekly, twice-weekly, biweekly, once-monthly, every other month, or at less frequent intervals. Preferably, the binding compositions are applied weekly or once per month to achieve the desired results.

Traditional methods of permanent hair waving, hair curling, or straightening use hydrogen peroxide to rebuild the disulfide bonds after a reducing treatment. The process generally takes about three days to complete. The methods disclosed herein use binding agents to repair the hair; these binding agents are washed from the individual's hair on the same day that they are applied to the hair. In some embodiments, the binding agents and the free thiol groups form a carbon-sulfur covalent bond. Under the same conditions, such as temperature and moisture, hair treated with the binding agents takes a longer time to revert to its prior state compared to the same hair that is untreated. The binding agent can contain one or more reactive groups where the reactive functional groups are bound to the surface.

In one embodiment, the binding agent contains a linker or spacer and two or more reactive functional groups, wherein the reactive functional groups are ionically bound to the linker or spacer. In other embodiments, the binding agent contains one or more reactive groups where the reactive functional groups interact with the surface of the hair or functional groups on the hair.

### DETAILED DESCRIPTION OF THE INVENTION

### I. Definitions

The term "hair" refers to one or more than one strand of hair, as well as the natural components of hair, such as oil from a body. Hair also refers to virgin hair or processed hair, for example hair that has been exposed to hair waving or hair straightening formulations.

An "effective amount", e.g., of the binding agent or compositions described herein, refers to an amount of the binding agent in a composition or formulation which, when applied as part of a desired dosage regimen, binds free thiols in the hair.

"Pharmaceutically acceptable" and "cosmetically acceptable" are used interchangeably and refer to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio. More specifically, pharmaceutically acceptable refers to a material, compound, or composition which is suitable for use in contact with the skin, scalp, or hair. Pharmaceutically acceptable materials are known to those of ordinary skill in the art.

"Shampoo", as used herein, generally refers to a liquid or semi-solid formulation applied to the hair that contains detergent or soap for washing the hair.

"Conditioner", as used herein, generally refers to a formulation (e.g., liquid, cream, lotion, gel, semi-solid) applied to the hair to soften the hair, smooth the hair, and/or change the sheen of the hair.

"Analog" and "Derivative" are used herein interchangeably and refer to a compound that possesses the same core as the parent compound, but differs from the parent compound in bond order, the absence or presence of one or more atoms and/or groups of atoms, and combinations thereof. The derivative can differ from the parent compound, for example, in one or more substituents present on the core, which may include one or more atoms, functional groups, or substructures. In general, a derivative can be imagined to be formed, at least theoretically, from the parent compound via chemical and/or physical processes.

"Electrophilic group" or "electrophilic moiety" are used interchangeably and refer to one or more functional groups or moieties that have an affinity for or attract electrons.

"Michael acceptor", as used herein, is a species of electrophilic groups or moieties that participates in nucleophilic addition reactions. The Michael acceptor can be or can contain an α,β-unsaturated carbonyl-containing group or moiety, such as a ketone. Other Michael acceptors include pi-bonds, such as double or triple bonds conjugated to other pi-bond containing electron withdrawing groups, such as nitro groups, nitrile groups, and carboxylic acid groups.

"Alkyl", as used herein, refers to the radical of saturated or unsaturated aliphatic groups, including straight-chain alkyl, alkenyl, or alkynyl groups, branched-chain alkyl, alkenyl, or alkynyl groups, cycloalkyl, cycloalkenyl, or cycloalkynyl (alicyclic) groups, alkyl substituted cycloalkyl, cycloalkenyl, or cycloalkynyl groups, and cycloalkyl substituted alkyl, alkenyl, or alkynyl groups. Unless otherwise indicated, a straight chain or branched chain alkyl has 30 or fewer carbon atoms in its backbone (e.g., C₁-C₃₀ for straight chain, C₃-C₃₀ for branched chain), more preferably 20 or fewer carbon atoms, more preferably 12 or fewer carbon atoms, and most preferably 8 or fewer carbon atoms. In some embodiments, the chain has 1-6 carbons. Likewise, preferred cycloalkyls have from 3-10 carbon atoms in their ring structure, and more preferably have 5, 6 or 7 carbons in the ring structure. The ranges provided above are inclusive of all values between the minimum value and the maximum value.

The term "alkyl" includes both "unsubstituted alkyls" and "substituted alkyls", the latter of which refers to alkyl moieties having one or more substituents replacing a hydrogen on one or more carbons of the hydrocarbon backbone. Such substituents include, but are not limited to, halogen, hydroxyl, carbonyl (such as a carboxyl, alkoxycarbonyl, formyl, or an acyl), thiocarbonyl (such as a thioester, a thioacetate, or a thioformate), alkoxyl, phosphoryl, phosphate, phosphonate, a phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, or an aromatic or heteroaromatic moiety.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths. Preferred alkyl groups are lower alkyls.

The alkyl groups may also contain one or more heteroatoms within the carbon backbone. Examples include oxygen, nitrogen, sulfur, and combinations thereof. In certain embodiments, the alkyl group contains between one and four heteroatoms.

"Alkenyl" and "Alkynyl", as used herein, refer to unsaturated aliphatic groups containing one or more double or triple bonds analogous in length (e.g., C₂-C₃₀) and possible substitution to the alkyl groups described above.

"Aryl", as used herein, refers to 5-, 6- and 7-membered aromatic rings. The ring may be a carbocyclic, heterocyclic, fused carbocyclic, fused heterocyclic, bicarbocyclic, or biheterocyclic ring system, optionally substituted as described above for alkyl. Broadly defined, "Ar", as used herein, includes 5-, 6- and 7-membered single-ring aromatic groups that may include from zero to four heteroatoms. Examples include, but are not limited to, benzene, pyrrole, furan, thiophene, imidazole, oxazole, thiazole, triazole, pyrazole, pyridine, pyrazine, pyridazine and pyrimidine. Those aryl groups having heteroatoms in the ring structure may also be referred to as "heteroaryl", "aryl heterocycles", or "heteroaromatics". The aromatic ring can be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, perfluoroalkyl, and cyano. The term "Ar" also includes polycyclic ring systems having two or more cyclic rings in which two or more carbons are common to two adjoining rings (the rings are "fused rings") wherein at least one of the rings is aromatic, e.g., the other cyclic rings can be cycloalkyls, cycloalkenyls, cycloalkynyls, aryls and/or heterocycles, or both rings are aromatic.

"Alkylaryl", as used herein, refers to an alkyl group substituted with an aryl group (e.g., an aromatic or hetero aromatic group).

"Heterocycle" or "heterocyclic", as used herein, refers to a cyclic radical attached via a ring carbon or nitrogen of a monocyclic or bicyclic ring containing 3-10 ring atoms, and preferably from 5-6 ring atoms, containing carbon and one to four heteroatoms each selected from non-peroxide oxygen, sulfur, and N(Y) wherein Y is absent or is H, O, (C₁₋₄) alkyl, phenyl or benzyl, and optionally containing one or more double or triple bonds, and optionally substituted with one or more substituents. The term "heterocycle" also encompasses substituted and unsubstituted heteroaryl rings. Examples of heterocyclic ring include, but are not limited to, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinriolinyl, decahydroquinolinyl, 2*H*,6*H*-1,5,2-dithiazinyl, dihydrofuro[2,3-*b*]tetrahydrofuranyl, furanyl, furazanyl, imidazolidinyl, imidazolinyl, imidazolyl, 1*H*-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl and xanthenyl.

"Heteroaryl", as used herein, refers to a monocyclic aromatic ring containing five or six ring atoms containing carbon and 1, 2, 3, or 4 heteroatoms each selected from non-peroxide oxygen, sulfur, and N(Y) where Y is absent or is H, O, (C₁-C₈) alkyl, phenyl or benzyl. Non-limiting examples of heteroaryl groups include furyl, imidazolyl, triazolyl, triazinyl, oxazoyl, isoxazoyl, thiazolyl, isothiazoyl, pyrazolyl, pyrrolyl, pyrazinyl, tetrazolyl, pyridyl, (or its N-oxide), thienyl, pyrimidinyl (or its N-oxide), indolyl, isoquinolyl (or its N-oxide), quinolyl (or its N-oxide) and the like. The term "heteroaryl" can include radicals of an ortho-fused bicyclic heterocycle of about eight to ten ring atoms derived therefrom, particularly a benz-derivative or one derived by fusing a propylene, trimethylene, or tetramethylene diradical thereto. Examples of heteroaryl include, but are not limited to, furyl, imidazolyl, triazolyl, triazinyl, oxazoyl, isoxazoyl, thiazolyl, isothiazoyl, pyraxolyl, pyrrolyl, pyrazinyl, tetrazolyl, pyridyl (or its N-oxide), thienyl, pyrimidinyl (or its N-oxide), indolyl, isoquinolyl (or its N-oxide), quinolyl (or its N-oxide), and the like.

"Halogen", as used herein, refers to fluorine, chlorine, bromine, or iodine.

The term "substituted" as used herein, refers to all permissible substituents of the compounds described herein. In the broadest sense, the permissible substituents include acyclic and cyclic, branched and unbranched, carbocyclic and heterocyclic, aromatic and nonaromatic substituents of organic compounds. Illustrative substituents include, but are not limited to, halogens, hydroxyl groups, or any other organic groupings containing any number of carbon atoms, preferably 1-14 carbon atoms, and optionally include one or more heteroatoms such as oxygen, sulfur, or nitrogen grouping in linear, branched, or cyclic structural formats. Representative substituents include alkyl, substituted alkyl, alkenyl, substituted alkenyl, alkynyl, substituted alkynyl, phenyl, substituted phenyl, aryl, substituted aryl, heteroaryl, substituted heteroaryl, halo, hydroxyl, alkoxy, substituted alkoxy, phenoxy, substituted phenoxy, aroxy, substituted aroxy, alkylthio, substituted alkylthio, phenylthio, substituted phenylthio, arylthio, substituted arylthio, cyano, isocyano, substituted isocyano, carbonyl, substituted carbonyl, carboxyl, substituted carboxyl, amino, substituted amino, amido, substituted amido, sulfonyl, substituted sulfonyl, sulfonic acid, phosphoryl, substituted phosphoryl, phosphonyl, substituted phosphonyl, polyaryl, substituted polyaryl, C₃-C₂₀ cyclic, substituted C₃-C₂₀ cyclic, heterocyclic, substituted heterocyclic, aminoacid, peptide, and polypeptide groups.

Heteroatoms, such as nitrogen, may have hydrogen substituents and/or any permissible substituents of organic compounds described herein that satisfy the valences of the heteroatoms. It is understood that "substitution" or "substituted" includes the implicit proviso that such substitution is in accordance with permitted valence of the substituted atom and the substituent, and that the substitution results in a stable compound, *i.e.* a compound that does not spontaneously undergo transformation such as by rearrangement, cyclization, elimination, etc.

"Polymer", as used herein, refers to a molecule containing more than 10 monomer units.

"Water-soluble", as used herein, generally means at least 10, 50, 100, 125, 150, 200, 225, or 250 g is soluble in 1L of water at 25°C.

"Binding agent", as used herein, means as used herein, refers to a molecule that forms covalent, ionic or hydrogen bonding, etc. with the hair and generally includes the formation of at least one covalent bond with a free thiol.

### II. Binding Formulations

The formulations disclosed herein are concerned with treating hair or skin. In particular, the formulations can rebuild latent disulfide bonds in hair or skin. Additionally, the formulations may also react with free amines in the hair to provide a conditioning effect.

The formulations contain one or more binding agents (also referred to herein as "compounds" or "active agents").

The binding agents can be combined with one or more pharmaceutically acceptable carriers and/or excipients that are considered safe and effective to human hair, skin, and/or human scalp, and may be administered to an individual's hair without causing undesirable side effects, such as burning, itching, and/or redness, or similar adverse reactions. The formulations may further contain an excipient that renders the formulations neutral pH, or a pH ranging from about pH 3 to about pH 12, preferably from pH 5 to pH 8.

The binding agent is typically present in an amount ranging from about 0.01 wt% to about 50 wt% of the formulation, preferably from about from about 1 wt% to about 25 wt% of the formulation, more preferably from about 1 wt% to about 15 wt%, most preferably from about 1 wt% to about 10 wt%. Typically, the binding agent is about 2.5-3 wt% of the formulation.

The binding agent is stable in aqueous solution for a period of at least 2, 3, 4, 5, 6, 8, 9, 10, 11, or 12 months or longer at pH of 6 to 8 and a temperature of about 25-30°C, preferably about 25°C. "Stable" as used herein with respect to shelf-life means that at least 40, 45, 50, 55, 60, 65, 70, 75, 80, 85, 90, or 95% of the reactive moieties are intact or to the extent that the reactive moieties react with water, the resulting product is also electrophilic.

### a. Binding agent

The binding agent contains at least two reactive moieties capable of reacting with a thiol. The binding agent optionally contains a linker between the two or more reactive moieties. The linker forms two or more ionic bonds with the reactive moieties. The reactive moieties, upon reaction with thiol groups on the hair follicle, form bonds that are stable, for example, hydrolytically stable. "Stable", as used in reference to the bonds formed between thiol groups on hair follicles means the bonds remain intact for at least one week, two weeks, three weeks, four weeks, one month, or two months or longer when exposed to water at pH 6-8 at a temperature from about 5°C to about 100°C, preferably from about 20°C to about 75°C, more preferably from about 20°C to about 50°C, more preferably from about 25°C to about 40°C, most preferably from about 25°C to about 30°C. In some embodiments, the temperature is about 25 °C. It is also preferred that the binding reaction occur around room temperature, for example, from about 15°C to about 35°C, preferably from about 20°C to about 30°C, more preferably from about 22°C to about 27°C.

The binding agents typically have a low molecular weight and are compatible with aqueous or solvent delivery systems. In some embodiments, the compound is water-soluble. The low molecular weight is preferred, as it allows the molecule to diffuse in and out of hair at a reasonable rate. Molecular weights of less than 10,000 Da, 8,000 Da, 6,000 Da, 5,000 Da, 4,000 Da, 3,000 Da, 2,000 Da, or 1,000 Da are preferred. In some embodiments, the molecular weight is less than 1500 Da, preferably less than 800 Da, most preferably less than 500 Daltons to achieve sufficient diffusion rates in conventional aqueous hair care systems.

### i. Binding agents defined by Formula I

The binding agents have a structure according to Formula I: 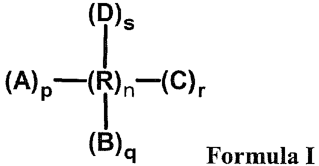 wherein
A, B, C, and D are reactive moieties capable of reacting with the free thiol groups each containing one or more charges, wherein each of the A, B, C and D reactive moieties independently contain a moiety selected from the group consisting of vinyl sulfone, an 30 acrylate group, a methacrylate group, a styrene group, an acryl amide group, a methacryl amide group, a maleate group, a fumarate group, and an itaconate group;
(R)ₙ is a linker that contains two or more charges, wherein the charges are opposite to the charges on the reactive moieties, wherein R is a monomer, n=1-10, and wherein (R)ₙ is not a polymer,
and the sum of the charges is zero, and wherein the reactive moieties are ionically bound to the linker;
   and
each occurrence of p, q, r, and s is independently an integer from 0 to 25, preferably from 0 to 10, more preferably from 0 to 2. The sum of p + q + r + s is equal to or greater than 2; provided that the binding agent of formula I is not a trimaleate salt of

   H₂N-(CH₂)₃-N¹(G)-(CH₂)₄-NH₂

   wherein G is a substituent that is bound to the secondary amine group of spermidine, chosen from saturated or unsaturated, linear or branched alkyl groups formed from 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene; aryl or aryl-alkyl groups, such as phenyl, naphthyl, benzyl, tolyl, wherein one or more carbon atoms are optionally replaced by fluorine, and wherein said aryl-alkyl groups include saturated or unsaturated, linear or branched alkyl groups formed by 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene; saturated or unsaturated cycloalkyl groups formed by 3 to 8 atoms of carbon that are optionally replaced by saturated or unsaturated, linear or branched alkyl groups formed by 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene.

The reactive moieties may be present on any atom of the linker. In some embodiments, the reactive moieties are the same. In some embodiments, one or more of the reactive moieties is different.

In some embodiments, the reactive moieties are negatively charged and the linker or spacer has positively charged moieties. In other embodiments, the reactive moieties are positively charged and the linker or spacer has negatively charged moieties.

### ii. Linker

The reactive moieties on the binding agents are linked via a linker (R)ₙ. The term "linker", as used herein, refers to one or more polyfunctional, e.g. bifunctional molecules, trifunctional molecules, tetrafunctional molecules, etc., which can be used to ionically bound the two or more reactive moieties and which do not interfere with the reactive properties of the binding agents. The reactive moieties may be attached to any part of the linker.

Linkers can be a single atom, such as a heteroatom (e.g., O or S), a group of atoms, such as a functional group (e.g., amine, -C(=O)-, -CH₂-), or multiple groups of atoms, such as an alkylene chain. Suitable linkers include but are not limited to oxygen, sulfur, carbon, boron, nitrogen, alkoxy, alkyl, alkenyl, cycloalkyl, cycloalkenyl, aryl, heterocycloalkyl, heteroaryl, ether and amine.

The linker is optionally independently substituted with one or more substituents including hydrogen, halogen, cyano, alkoxy, alkyl, alkenyl, cycloalkyl, cycloalkenyl, aryl, heterocycloalkyl, heteroaryl, amine, hydroxy, formyl, acyl, carboxylic acid (-COOH), -C(O)R¹, -C(O)OR¹, carboxylate (-COO⁻), primary amide (e.g., -CONH₂), secondary amide (e.g., -CONHR¹), -C(O)NR¹R², -NR¹R², -NR¹S(O)₂R², -NR¹C(O)R², -S(O)₂R², -SR¹, and -S(O)₂NR¹R², sulfinyl group (e.g., -SOR¹), and sulfonyl group (e.g., -SOOR¹); wherein R¹ and R² may each independently be hydrogen, alkyl, alkenyl, alkynyl, cycloalkyl, aryl, heterocycloalkyl and heteroaryl; wherein each of R¹ and R² is optionally independently substituted with one or more substituents selected from the group consisting of halogen, hydroxyl, cyano, nitro, amino, alkylamino, dialkylamino, alkyl optionally substituted with one or more halogen or alkoxy or aryloxy, aryl optionally substituted with one or more halogen or alkoxy or alkyl or trihaloalkyl, heterocycloalkyl optionally substituted with aryl or heteroaryl or =O or alkyl optionally substituted with hydroxyl, cycloalkyl optionally substituted with hydroxyl, heteroaryl optionally substituted with one or more halogen or alkoxy or alkyl or trihaloalkyl, haloalkyl, hydroxyalkyl, carboxy, alkoxy, aryloxy, alkoxycarbonyl, aminocarbonyl, alkylaminocarbonyl and dialkylaminocarbonyl.

In some embodiments, the linker may be an alkoxy, ether, alkyl, alkenyl, cycloalkyl, cycloalkenyl, aryl, heterocycloalkyl, heteroaryl or an amine. The linker is not a polymer.

### iv. Reactive moieties that react with thiols

The binding agent contains at least two reactive moieties that react with thiols to form covalent bonds. The reactive moieties are capable of reacting with a thiol group in the hair or on the skin to form a stable covalent bond. The reactive moiety is typically an electrophilic moiety capable of forming a salt with the linker.
Alternately, the reactive moiety can be a free radical forming moiety.

The binding agent contains at least two reactive moieties. However, the binding agent may contain three, four, five, six, or greater than six reactive moieties.

The reaction between the reactive moiety and the thiol groups may be initiated at room temperature and pressure when the reactive moiety contacts a thiol group in the hair or on the skin. In some embodiments, the reaction may require an initiator, such as heat, catalyst, basic conditions, or a free radical initiator. The rate of reaction between the reactive moiety and the thiol may be increased by changes in temperature, pH, and/or addition of one or more excipients, such as a catalyst;
however, this is generally not required.

The two or more reactive moieties on the binding agent can be the same. In some embodiments, the two or more reactive moieties are different.

In some embodiments, the reactive moieties are capable of undergoing a conjugate additional reaction. The reactive moieties can independently be or contain a vinyl sulfone, an acrylate group, a methacrylic or methacrylate group, a styrene group, an acryl amide group, a methacryl amide group, a maleate group, a fumarate group or an itaconate group.

### Vinyl sulfone

The chemistry of vinyl sulfones with respect to attack by nucleophiles is analogous to that of α,β-unsaturated ketones in that they can undergo a 1,4- type Michael addition without releasing any undesirable by-products.

### Free radical-forming groups

The binding agent may contain at least two free radical-forming groups that can react with thiols. The free radical-forming groups on the binding agent can be the same. Alternately, the free radical-forming groups may be different. Suitable free radical forming groups include acrylate groups, methacrylate groups, styrene groups, acryl amide groups, methacryl amide groups, maleate groups, fumarate groups and itaconate groups. For example, suitable binding agents include ethylene glycol dimethacrylate, diethylene glycol diacrylate, allyl methacrylate, trimethylolpropane triacrylate and di- and triacrylates, mixed acrylates which, as well as acrylate groups, comprise further ethylenically unsaturated groups. Other examples of binding agents include N,N'-methylenebisacrylamide and N,N'-methylenebismethacrylamide, esters of unsaturated mono- or polycarboxylic acids of polyols, such as diacrylate or triacrylate, for example butanediol diacrylate, butanediol dimethacrylate, ethylene glycol diacrylate, ethylene glycol dimethacrylate and also trimethylolpropane triacrylate and allyl compounds, such as allyl (meth)acrylate, and diallyl maleate.
Other examples of binding agents include di- and triacrylates of 3- to 15-tuply ethoxylated glycerol, of 3- to 15-tuply ethoxylated trimethylolpropane, of 3- to 15-tuply ethoxylated trimethylolethane, especially di- and triacrylates of 2- to 6- tuply ethoxylated glycerol or of 2- to 6-tuply ethoxylated trimethylolpropane, of 3-tuply propoxylated glycerol, of 3-tuply propoxylated trimethylolpropane, and also of 3-tuply mixed ethoxylated or propoxylated glycerol, of 3-tuply mixed ethoxylated or propoxylated trimethylolpropane, of 15-tuply ethoxylated glycerol, of 15-tuply ethoxylated trimethylolpropane, of 40-tuply ethoxylated glycerol, of 40-tuply ethoxylated trimethylolethane and also of 40-tuply ethoxylated trimethylolpropane, ethylene glycol dimethacrylate, diethylene glycol diacrylate, allyl methacrylate, trimethylolpropane triacrylate, N,N¹- methylenebisacrylamide, N,N'-methylenebismethacryl amide, butanediol diacrylate, butanediol dimethacrylate, trimethylolpropane triacrylate, triallyl cyanurate, diallyl maleate, di- and tri-acrylates of 3- to 15-tuply ethoxylted glycerol, di- and tri-acrylates of 3- to 15-tuply ethoxylated trimethylolpropane, and di- and tri-acrylates of 3- to 15-tuply ethoxylated trimethylolethane. As used herein, the term "tuply" refers to the number of monomeric units in the ethoxylated chain.

The reactive free radical moieties may require the presence of one or more initiators. Suitable initiators include, but are not limited to peroxides, hydroperoxides, hydrogen peroxide, persulfates, azo compounds, and redox initiators. Suitable organic peroxides include acetylacetone peroxide, methyl ethyl ketone peroxide, tert-butyl hydroperoxide, cumene hydroperoxide, tert-amyl perpivalate, tert-butyl perpivalate, tert-butyl perneohexanoate, tert-butyl perisobutyrate, tert-butyl per-2-ethylhexanoate, tert-butyl perisononanoate, tert-butyl permaleate, tert-butyl perbenzoate, di(2-ethylhexyl) peroxydicarbonate, dicyclohexyl peroxydicarbonate, di(4-tert-butylcyclohexyl) peroxydicarbonate, dimyristil peroxydicarbonate, diacetyl peroxydicarbonate, allyl peresters, cumyl peroxyneodecanoate, tert-butyl per-3,5,5-trimethylhexanoate, acetylcyclohexylsulfonyl peroxide, dilauryl peroxide, dibenzoyl peroxide, and tert-aryl perneodecanoate. Suitable azo compounds include 2,2'-azobisisobutyronitrile, 2,2'-azobis(2,4-dimethylvaleronitrile) and 2,2'-azobis(4-methoxy-2,4-dimethylvaleronitrile), preferably water-soluble azo initiators, such as, but not limited to, 2,2'-azobis{2-[1-(2-hydroxyethyl)-2-imidazolin-2-yl]propane}dihydrochloride, 2,2'-azobis-(2-amidinopropane)dihydrochloride, 2,2'-azobis[2-(2-imidazolin-2-yl)propane]dihydrochloxide and 2,2'-azobis[2-(5-methyl-2-imidazolin-2-yl)propane]dihydrochloride. For the redox initiators, the oxidizing component is at least one of the peroxo compounds indicated above and the reducing component is, for example, ascorbic acid, glucose, sorbose, ammonium bisulfite, ammonium sulfite, ammonium thiosulfate, ammonium hyposulfite, ammonium pyrosulfite, ammonium sulfide, alkali metal bisulfite, alkali metal sulfite, alkali metal thiosulfate, alkali metal hyposulfite, alkali metal pyrosulfite, alkali metal sulfide, or sodium hydroxymethylsulfoxylate.

In some embodiments, the molecule is: 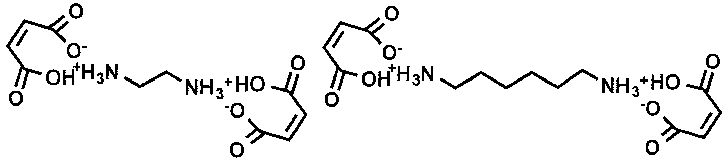 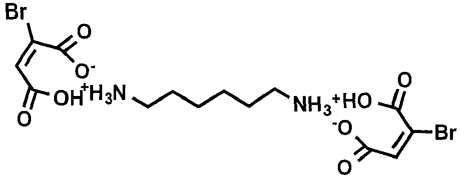 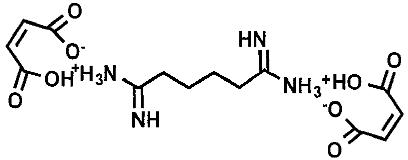 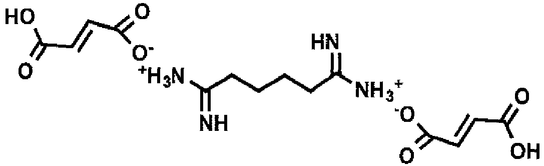 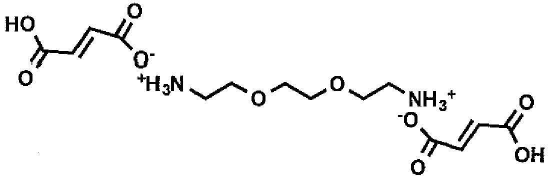 or

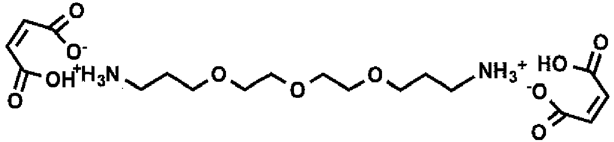

In some embodiments, the binding agent has the structure:

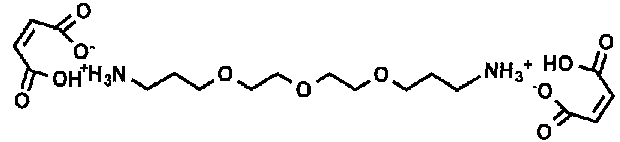

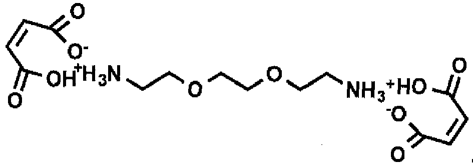

The reaction with thiol groups on hair follicles is as follows:

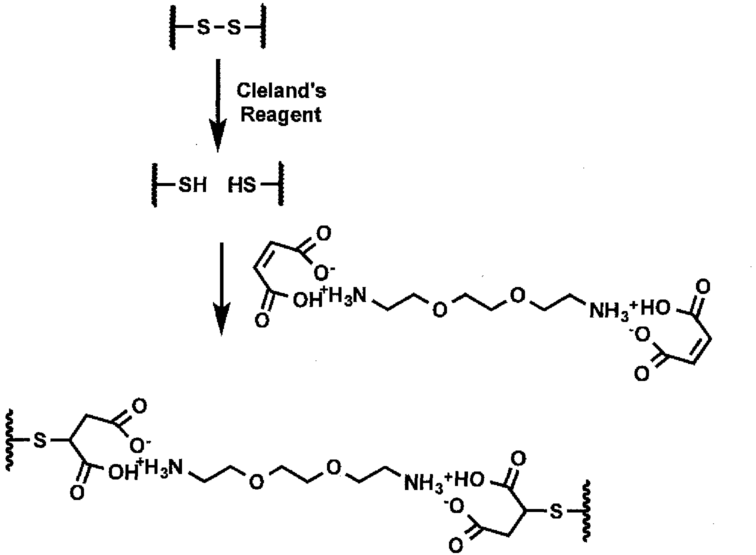

Other agents include, but are not limited to, acid-containing electrophiles, such as acrylic acid and bromo-acetic acid and similar compounds.

### b. Excipients

The formulations typically contain one or more cosmetically acceptable excipients. Cosmetically acceptable excipients include, but are not limited to, water, preservatives, antioxidants, chelating agents, sunscreen agents, vitamins, dyes, hair coloring agents, proteins, amino acids, natural extracts such as plant extracts, humectants, fragrances, perfumes, oils, emollients, lubricants, butters, penetrants, thickeners, viscosity modifiers, polymers, resins, hair fixatives, film formers, surfactants, detergents, emulsifiers, opacifying agents, volatiles, propellants, liquid vehicles, carriers, salts, pH adjusting agents (e.g., citric acid), neutralizing agents, buffers, hair conditioning agents, anti-static agents, anti-frizz agents, anti-dandruff agents, absorbents, and combinations thereof.

The formulations can contain at least two or more cosmetically acceptable excipients. In some forms, the formulations contain the binding agent, water, and optionally a preservative and/or fragrance.

The formulation for treating hair may be in any suitable physical form. Suitable forms include, but are not limited to low to moderate viscosity liquids, lotions, milks, mousses, sprays, gels, creams, shampoos, conditioners, and the like. Suitable excipients, such as those listed above, are included or excluded from the hair care formulation depending on the form of use of the formulation (*e.g*., hair spray, cream, conditioner, or shampoo).

The formulation for treating skin may be in any suitable physical form. Suitable forms include, but are not limited to low to moderate viscosity liquids, lotions, milks, mousses, sprays, gels, creams, ointments, and the like. Suitable excipients, such as those listed above, are included or excluded from the skin formulation depending on the form of use of the formulation (*e.g*., lotion, gel, ointment, or cream).

The pharmaceutical excipient is typically present in an amount ranging from about 10 wt% to about 99.99 wt% of the formulation, preferably about 40 wt% to about 99 wt%, more preferably from about 80 wt% to about to about 99 wt%.

### i. Surfactants

Surfactants are surface-active agents that are able to reduce the surface tension of water and cause the formulation to slip across or onto the skin or hair. Surfactants also include detergents and soap. The surfactants may be amphoteric, anionic, or cationic. Suitable surfactants that may be used in the formulation include, but are not limited to, 3-aminopropane sulfonic acid, almond amide, almond amidopropyl betaine, almond amidopropylamine oxide, aluminum hydrogenated tallow glutamate, aluminum lanolate, aminoethyl sulfate, aminopropyl lauryl glutamine, ammonium C₁₂₋₁₅ alkyl sulfate, ammonium C₁₂₋₁₅ pareth sulfate, ammonium C₁₂₋₁₆ alkyl sulfate, ammonium C₉₋₁₀ perfluoroalkylsulfonate, ammonium capryleth sulfate, ammonium capryleth-3 sulfate, ammonium monoglyceride sulfate, ammonium sulfate, ammonium isothionate, ammonium cocoyl sarcosinate, ammonium cumene sulfonate, ammonium dimethicone copolyol sulfate, ammonium dodecylbenzenesulfonate, ammonium isostearate, ammonium laureth sulfate, ammonium laureth-12 sulfate, ammonium laureth-5 sulfate, ammonium laureth-6 carboxylate, ammonium laureth-7 sulfate, ammonium laureth-8 carboxylate, ammonium laureth-9 sulfate, ammonium lauroyl sarcosinate, ammonium lauryl sulfate, ammonium lauryl sulfosuccinate, ammonium myreth sulfate, ammonium myristyl sulfate, ammonium nonoxynol-30 sulfate, ammonium nonoxynol-4 sulfate, ammonium oleate, ammonium palm kernel sulfate, ammonium polyacrylate, ammonium stearate, ammonium tallate, ammonium xylene sulfonate, ammonium xylene sulfonate, amp-isostearoyl gelatin/keratin amino acids/lysine hydroxypropyltrimonium chloride, amp-isostearoyl hydrolyzed collagen, apricot kernel oil PEG-6 esters, apricot amide, apricot amidopropyl betaine, arachideth-20, avocadamide, avocadamidopropyl betaine, babassuamide, babassuamidopropyl betaine, babassuamidopropylamine oxide, behenalkonium chloride, behenamide, behenamide, behenamidopropyl betaine, behenamine oxide, sodium laureth sulfate, sodium lauryl sulfate, a polyoxyether of lauryl alcohol or ceteareth-20, or combinations thereof.

Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer 401, stearoyl monoisopropanolamide, and polyoxyethylene hydrogenated tallow amide. Examples of amphoteric surfactants include sodium N-dodecyl-.beta.-alanine, sodium N-lauryl-β-iminodipropionate, myristoamphoacetate, lauryl betaine and lauryl sulfobetaine.

More than one surfactant may be included in the formulation.

The surfactants are optionally included in an amount ranging from about 0.1% to about 15% by weight of the formulation, preferably about 1% to about 10% by weight of the formulation.

### ii. Emollients

Emollient refers to a material that protects against wetness or irritation, softens, soothes, coats, lubricates, moisturizes, protects, and/or cleanses the skin. Suitable emollients for use in the formulations include, but are not limited to a silicone compound (*e.g*., dimethicone, cyclomethicone, dimethicone copolyol or a mixture of cyclopentasiloxane and dimethicone/vinyldimethicone cross polymer, cyclopentasiloxane polysilicone), polyols such as sorbitol, glycerin, propylene glycol, ethylene glycol, polyethylene glycol, caprylyl glycol, polypropylene glycol, 1,3-butane diol, hexylene glycol, isoprene glycol, xylitol; ethylhexyl palmitate; a triglyceride such as caprylic/capric triglyceride and fatty acid ester such as cetearyl isononanoate or cetyl palmitate. In a specific embodiment, the emollient is dimethicone, amidodimethicone, dimethiconol, cyclopentasiloxane, potassium dimethicone PEG-7 panthenyl phosphate, or a combination thereof. More than one emollient may be included in the formulation.

The emollient is optionally included in an amount ranging from about 0.5% to about 15% by weight of the formulation, preferably from about 1% to about 10% by weight of the formulation.

### iii. Emulsifiers

The formulations may also contain one or more emulsifiers. Suitable emulsifiers include, but are not limited to, copolymers of an unsaturated ester and styrene sulfonate monomer, cetearyl alcohol, glyceryl ester, polyoxyethylene glycol ether of cetearyl alcohol, stearic acid, polysorbate-20, ceteareth-20, lecithin, glycol stearate, polysorbate-60, or polysorbate-80, or combinations thereof. More than one emulsifier may be included in the formulation.

The emulsifier is optionally included in an amount ranging from about 0.05% to about 15% by weight of the formulation, preferably from about 0.1% to about 10% by weight of the formulation.

### iv. Preservatives

One or more preservatives may be included in the formulations to prevent microbial growth in the formulations. Suitable preservatives include, but are not limited to, glycerin containing compounds (*e.g.*, glycerin or ethylhexylglycerin or phenoxyethanol), benzyl alcohol, parabens (methylparaben, ethylparaben, propylparaben, butylparaben, isobutylparaben, etc.), sodium benzoate, ethylenediamine-tetraacetic acid (EDTA), potassium sorbate, and/or grapefruit seed extract, or combinations thereof. More than one preservative may be included in the formulation. Other preservatives are known in the cosmetics industries and include salicylic acid, DMDM Hydantoin, Formaldahyde, Chlorphenism, Triclosan, Imidazolidinyl Urea, Diazolidinyl Urea, Sorbic Acid, Methylisothiazolinone, Sodium Dehydroacetate, Dehydroacetic Acid, Quaternium -15, Stearalkonium Chloride, Zinc Pyrithione, Sodium Metabisulfite, 2-Bromo-2-Nitropropane, Chlorhexidine Digluconate, Polyaminopropyl biguanide, Benzalkonium Chloride, Sodium Sulfite, Sodium Salicylate, Citric Acid, Neem Oil, Essential Oils (various), Lactic Acid, and Vitamin E (tocopherol).

The preservative is optionally included in an amount ranging from about 0.1 % to about 5% by weight of the formulation, preferably from about 0.3% to about 3% by weight of the formulation. Preferably, the formulations are paraben free.

### v. Conditioning Agents

One or more conditioning agents may be included in the formulations. Suitable conditioning agents include, but are not limited to, silicone-based agents (e.g., silicone quaternium-8), panthenol, hydrolyzed wheat and/or soy protein, amino acids (*e.g.* wheat amino acids), rice bran wax, meadowfoam seed oil, mango seed oil, grape seed oil, jojoba seed oil, sweet almond oil, hydroxyethyl behenamidopropyl dimonium chloride, aloe leaf extract, aloe barbadensis leaf juice, phytantriol, panthenol, retinyl palmitate, behentrimonium methosulfate, cyclopentasiloxane, quaternium-91, stearamidopropyl dimethylamine, and combinations thereof.

The conditioning agent(s) is optionally included in an amount ranging from about 0.1% to about 5% by weight of the formulation, preferably from about 0.3% to about 3% by weight of the formulation.

### vi. Diluents

Diluent, as used herein, refers to a substance(s) that dilutes the binding agent. Water is the preferred diluent. The formulations typically contains greater than one percent (wt) water, preferably greater than five percent (wt) water, more preferably greater than 50% (wt) water, and most preferably greater than 80% (wt)water. Alcohols, such as ethyl alcohol and isopropyl alcohol, may be used at low concentrations (about 0.5% by weight of the formulation) to enhance hair or skin penetration and/or reduce odor.

### vii. Viscosity modifying agents

The formulations may contain one or more viscosity modifying agents, such as viscosity increasing agents. Classes of such agents include, but are not limited to, viscous liquids, such as polyethylene glycol, semisynthetic polymers, such as semisynthetic cellulose derivatives, synthetic polymers, such as carbomers, poloxamers, and polyethyleneimines (e.g., PEI-10), naturally occurring polymers, such as acacia, tragacanth, alginates (e.g., sodium alginate), carrageenan, vegetable gums, such as xanthan gum, petroleum jelly, waxes, particulate associate colloids, such as bentonite, colloidal silicon dioxide, and microcrystalline cellulose, surfactants, such as PPG-2 hydroxyethyl coco/isostearamide, emulsifiers, such as disteareth-75 IPDI, and salts, such as sodium chloride, and combinations thereof.

### viii. Antioxidants

The formulations may contain one or more antioxidants. Examples include, but are not limited to, tocopheryls, BHT, ascorbic acid, camellia sinensis leaf extract, ascorbyl palmitate, magnesium ascorbyl phosphate, carotenoids, resveratrol, triethyl citrate, arbutin, kojic acid, tetrahexydecyl ascorbate, superoxide dismutase, zinc, sodium metabisulfite, lycopene, ubiquinone, and combinations thereof.

### ix. Opacifying agents

The formulations may contain one or more opacifying agents. Opacifying agents are added to the formulations to make them opaque. Suitable opacifying agents include, but are not limited to, glycol distearate and ethoxylated fatty alcohols.

### c. Forms of the formulation

### i. Sprays

The formulation may be in the form of a spray. The spray typically includes the binding agent and a cosmetically acceptable carrier. In some embodiments, the carrier is water or a water and alcohol mixture. The spray formulation optionally includes an antioxidant, sunscreen agent, vitamin, protein, peptide, plant extract, humectant, oil, emollient, lubricant, thickener, hair conditioning agent, polymer, and/or surfactant. Preferably, the spray formulation includes a preservative. In some embodiments, the formulation includes a fragrance. In some embodiments, the formulation includes a surfactant. In some embodiments, the formulation contains water, fragrance, a preservative, and a binding agent. In some embodiments, the formulation contains water, fragrance, a preservative, and a binding agent. In some embodiments, the formulation contains water, a preservative, fragrance, the binding agent, and an anti-static agent. In some embodiments, the formulation contains water, a preservative, fragrance, the binding agent, and a hair conditioning agent. In some embodiments, the formulation contains water, a preservative, fragrance, the binding agent, and a surfactant.

The hair spray formulations may be dispensed from containers that include aerosol dispensers or pump spray dispensers. Such dispensers are known in the art and are commercially available from a variety of manufacturers.

### Propellant

When the hair spray formulation is dispensed from a pressurized aerosol container, a propellant may be used to force the composition out of the container. Suitable propellants include, but are not limited to, a liquefiable gas or a halogenated propellant. Examples of suitable propellants include dimethyl ether and hydrocarbon propellants such as propane, n-butane, iso-butane, CFCs, and CFC-replacement propellants. The propellants may be used singly or admixed.

The amount of propellant may range from about 10% to about 60% by weight of the formulation. The propellant may be separated from the hair repair formulation as in a two compartment container. Other suitable aerosol dispensers are those characterized by the propellant being compressed air, which can be filled into the dispenser using a pump or equivalent device prior to use. Conventional non-aerosol pump spray dispensers, *i.e*., atomizers, may also be used to apply the hair strengthening formulation to the hair.

### ii. Conditioners

The formulation may be in the form of a conditioner. The conditioner typically includes the binding agent in a suitable carrier. Additionally, the conditioner may include cationic polymers derived from polysaccharides, for example cationic cellulose derivatives, cationic starch derivatives, cationic guar derivatives and cationic locust bean gum derivatives, synthetic cationic polymers, mixtures or combinations of these agents. The formulation may comprise other synthetic or natural polymers or polymers derived from biological preparation processes, which are functionalized, where appropriate, for example with cationic or neutral groups. These polymers may have a stabilizing or strengthening action on the compositions, and/or a conditioning action (deposition on the surface of the skin or the hair).

The binding agent may be included in any suitable concentration. Typical concentrations of the binding agent in the conditioner range from small amounts such as approximately 0.01% (wt), preferably at least 0.1% (wt), to large amounts, such as up to 50% (wt). Preferably the conditioner contains the binding agent in a concentration ranging from 0.1% (wt) to 5% (wt), more preferably from 0.1 % wt to 3% (wt). While greater concentrations of binding agent could be present in the conditioner, they are generally not needed to achieve the desired results.

### iii. Shampoos

The hair repair formulation may be in the form of a shampoo. The shampoo typically includes the binding agent in a suitable carrier. The binding agent may be included in any suitable concentration. Typical concentrations of the binding agent in the shampoo range from small amounts such as approximately 0.01% (wt), preferably at least 0.1 % (wt), to large amounts, such as up to 50% (wt). Preferably the shampoo contains the binding agent in a concentration ranging from 0.1% (wt) to 5% (wt), more preferably from 0.1% wt to 3% (wt). While greater concentrations of binding agent could be present in the shampoo, they are generally not needed to achieve the desired results.

Additionally, the shampoo may include from about 0.5% to about 20% of a surfactant material. Surfactants utilized in shampoo compositions are well-known in the art and are disclosed, for example, in U.S. Patent No. 6,706,258 to Gallagher et al. and U.S. Patent No. 7,598,213 to Geary et al.

### iv. Creams

The formulation may be in the form of a cream. The cream typically includes the binding agent in a suitable carrier. The binding agent may be included in any suitable concentration. Typical concentrations of the binding agent in the cream range from small amounts such as approximately 0.01% (wt), preferably at least 0.1% (wt), to large amounts, such as up to 50% (wt). Preferably the cream contains the binding agent in a concentration ranging from 0.1% (wt) to 5% (wt), more preferably from 0.1% wt to 3% (wt). While greater concentrations of binding agent could be present in the cream, they are generally not needed to achieve the desired results.

Additionally, the cream may include an oil, a hair conditioning agent, and/or a thickening agent. The cream may also include a fragrance, a plant extract, and/or a surfactant. The cream may be packaged in a tube, tub, bottle, or other suitable container.

### v. Liquid Binding Formulations

In some embodiments, a liquid binding formulation is provided, which is mixed at the time of use with a second formulation, such as a coloring or highlighting formulation. In these embodiments, the liquid binding formulation may contain any suitable concentration of binding agent in a suitable carrier, typically a diluent, such as described above. The concentration of the binding agent is suitable to provide a mixture with the appropriate final volume and final concentration of binding agent.

For example, a liquid binding formulation can contain a concentration of binding agent ranging from about 5% (wt) to about 50% (wt) or greater. In a preferred embodiment, the liquid binding formulation contains about 20% (wt) binding agent.

The terms "highlighting" and "bleaching" are used synonymously herein. For highlighting applications, prior to use, a sufficient volume of a liquid binding formulation is mixed with a sufficient volume of a highlighting formulation to form a highlighting mixture having the desired concentration of binding agent. Typical concentrations of the binding agent in the highlighting mixture range from small amounts, such as approximately at least 0.01 % (wt), preferably at least 0.1 % (wt), to large amounts, such as up to 50% (wt). Preferably the highlighting mixture contains the binding agent in a concentration ranging from 0.1 % (wt) to 5% (wt), more preferably from 0.1 % wt to 3% (wt). While greater concentrations of binding agent could be present in the highlighting mixture, they are generally not needed to achieve the desired results.

Alternatively, two separate formulations are applied, such as a first formulation containing bleach (i.e. the highlighting formulation), and a second formulation containing a binding agent (i. e. the binding formulation) in an effective amount to covalently bind the free thiol groups. The highlighting formulation may be applied first, which produces free thiol groups in hair. Subsequently, the second binding formulation may be applied to bind the free thiol groups.

### III. Kit

Kits for treating hair typically contain a binding formulation containing an effective amount of a binding agent to covalently bind latent free thiol groups in hair.

Instructions for use of the kit are also typically provided.

The present invention provides a kit comprising:
(a) a first formulation comprising a reducing agent capable of reducing disulfide bonds in hair to produce free thiol groups, and
(b) a second formulation comprising a binding agent in an effective amount to covalently bind free thiol groups in hair,
wherein the binding agent is represented by Formula I: 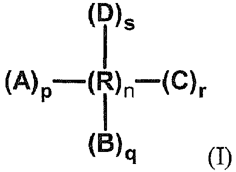 wherein
A, B, C, and D are reactive moieties capable of reacting with the free thiol groups each containing one or more charges, wherein each of the A, B, C, and D reactive moieties independently contain a moiety selected from the group consisting of a vinyl sulfone, an acrylate group, a methacrylate group, a styrene group, an acryl amide group, a methacryl amide group, a maleate group, a fumarate group, and an itaconate group;
(R)ₙ is a linker that contains two or more charges, wherein the charges are opposite to the charges on the reactive moieties, wherein R is a monomer, n = 1-10, and wherein (R)ₙ is not a polymer, and the sum of the charges is zero, and
wherein the reactive moieties are ionically bound to the linker;
wherein each occurrence of p, q, r, and s is independently an integer from 0 to 25, and wherein the sum of p + q + r + s is equal to or greater than 2.

The kit may further contain a formulation, also referred to herein as the reducing formulation, capable of reducing the disulfide bonds in the hair and producing free thiol groups.

### a. Reducing Formulation

The first formulation may be a reducing formulation. A reducing formulation contains a reducing agent capable of reducing the disulfide bonds in hair and producing free thiol groups. The reducing formulation may differ depending on the hair styling treatment desired (such as hair waving or hair straightening), the texture of the hair, the sensitivity of the user's skin, and the like.

Formulations containing reducing agents and their selection are well known to those skilled in the cosmetic industry. Suitable reducing agents include, but are not limited to, thioglycolic acid and thioglycolic acid salts and esters, thiolactic acid and thiolactic acid salts and esters, cysteine thioglycerol, thioglycolic hydrazide, thioglycolamide, glycerol monothioglycolate, sodium metabisulfite, beta-mercaptopropionic acid, N-hydroxyethyl mercapto-acetamide, N-methyl mercapto-acetamide, beta-mercapto-ethylamine, beta-mercaptopropionamide, 2-mercapto-ethanesulfonic acid, dimercaptoadipic acid, dithiothreitol, homocysteinethiolactone, cysteine derivatives, polythiol derivatives formed by the addition of cysteamine onto a maleic anhydride-alkylvinylether copolymer, inorganic sulfites, inorganic bisulfites, cysteamine and its derivatives, dithioerythritol, organic phosphines, and Japanese relaxers.

In some embodiments, the kit contains a reducing formulation, which contains a reducing agent for permanent hair waving and hair curling such as acid perms, alkaline perms, perms having neutral pH, or perms using buffered alkaline waving lotions. Such reducing agents include, but are not limited to thioglycolic acid and its derivative salts and esters, thiolactic acid and its derivative salts and esters, cysteine and its derivatives, cysteamine and its derivatives, inorganic sulfites, and inorganic bisulfites such as sodium metabisulfite, dithiothreitol, dithioerythritol, organic phosphines, and Japanese relaxers.

In other embodiments, the kit contains a reducing formulation, which contains a reducing agent for straightening hair. Such reducing agents include, but are not limited, to inorganic bisulfites such as sodium metabisulfite, inorganic sulfites, and ammonium thioglycolate, dithiothreitol, dithioerythritol, organic phosphines, and Japanese relaxers.

The amount of the reducing agent in the reducing formulation is sufficient to rupture a sufficient number of disulfide bonds for effective hair waving, hair curling, or hair straightening as would be appreciated by one of skill in the art.

### b. Coloring formulation

The first formulation may be a coloring treatment. The first formulation may be formulated as two or more components may be mixed together before application to the hair. For example, the first formulation may be in the form of two components such as a dye precursor and an oxidant. Typically, the hair coloring formulation contains a reducing agent capable of reducing the disulfide bonds in hair and producing free thiol groups. Suitable reducing agents include, but are not limited to, thioglycolic acid, thiolactic acid, dihydrolipoate, thioglycerol, mercaptopropionic acid, sodium bisulfite, ammonium bisulfide, zinc formaldehyde sulfoxylate, sodium formaldehyde sulfoxylate, sodium metabisulfite, potassium borohydride, pegylated thiols and hydroquinone. The amount of the reducing agent in the first formulation is sufficient to rupture a sufficient number of disulfide bonds for effective diffusion of the hair coloring ingredients as would be appreciated by one of skill in the art.

The components of the first formulation may differ depending on the hair coloring treatment desired (such as for semi-permanent, demi-permanent, or permanent hair color), the texture of the hair, the sensitivity of the user's skin, and such the like. Hair coloring formulations for different hair coloring treatment, hair texture, and hair sensitivity are known to those of skill in the art.

### c. Binding formulation

The binding formulation contains an effective amount of a binding agent to bind free thiols in the hair. Suitable formulations containing the binding agents are discussed above. The binding formulation may be in any suitable form. Suitable forms include, but are not limited to, low to moderate viscosity liquids, lotions, milks, mousses, sprays, gels, creams, shampoos, conditioners, and the like. The binding formulation will be present in a suitable container, which depends on the form of the formulation.

In one embodiment, the binding formulation is provided as two or more separate ingredients. For example, the binding agent may be provided as a dry powder in a sealed package and the excipient provided in a vial or other container. A suitable mixing container for the binding agent and the excipient may be provided.

Optionally, the binding agent is premixed with a shampoo or conditioner.
In some embodiments, the binding formulation (or second formulation) is mixed with the first formulation (reducing formulation or hair coloring treatment), and the mixture is applied to the hair.

### c. Other materials in the kit

The kit optionally contains shampoos and conditioners. Suitable shampoos and conditioners include, but are not limited to LiQWd® Hydrating Shampoo and LiQWd® Hydrating Conditioner.

The kit may further contain an odor eliminator. The odor eliminator can be incorporated into the reducing formulation. Alternately, the odor eliminator is present in a suitable container for use before or after washing the binding formulation from the hair. Some suitable odor eliminators are known to those of ordinary skill in the art.

### IV. Methods of Use

The present invention provides a method for treating hair, wherein the hair comprises two or more free thiol groups, comprising:
(a) applying to the hair a formulation comprising a binding agent of Formula I 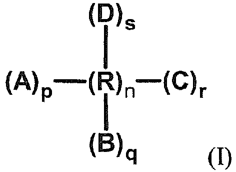 wherein
   A, B, C, and D are reactive moieties capable of reacting with the free thiol groups each containing one or more charges, wherein each of the A, B, C, and D reactive moieties independently contain a moiety selected from the group consisting of a vinyl sulfone, an acrylate group, a methacrylate group, a styrene group, an acryl amide group, a methacryl amide group, a maleate group, a fumarate group, and an itaconate group;
   (R)ₙ is a linker that contains two or more charges, wherein the charges are opposite to the charges on the reactive moieties, wherein R is a monomer, n = 1-10, and wherein (R)ₙ is not a polymer, and
   the sum of the charges is zero, and
   wherein the reactive moieties are ionically bound to the linker;
   wherein each occurrence of p, q, r, and s is independently an integer from 0 to 25, and wherein the sum of p + q + r + s is equal to or greater than 2, in an effective amount to covalently bind the free thiol groups;
   provided that the binding agent of formula I is not a trimaleate salt of

      H₂N-(CH₂)₃-N¹(G)-(CH₂)₄-NH₂

      wherein G is a substituent that is bound to the secondary amine group of spermidine, chosen from saturated or unsaturated, linear or branched alkyl groups formed from 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene; aryl or aryl-alkyl groups, such as phenyl, naphthyl, benzyl, tolyl, wherein one or more carbon atoms are optionally replaced by fluorine, and wherein said aryl-alkyl groups include saturated or unsaturated, linear or branched alkyl groups formed by 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene; saturated or unsaturated cycloalkyl groups formed by 3 to 8 atoms of carbon that are optionally replaced by saturated or unsaturated, linear or branched alkyl groups formed by 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene.

The methods disclosed herein are concerned with treating hair with free thiol groups.

### A. Treating damaged hair with free thiol groups

In one embodiment, prior to treatment with a binding agent, the hair has been damaged and the thiol groups in the hair are free thiols. The binding agent can be applied to the hair to bind the free thiol groups. Preferably, the binding agent is applied at least within one week of the hair being damaged, preferably within three days, more preferably within two days, most preferably, the same day.

### a. Rinse or wash the hair

Optionally, the hair may be shampooed and/or conditioned prior to applying the binding formulation. Alternately, the hair may only be rinsed with water prior to application of the binding formulation.

### b. Apply the binding formulation to the hair

Subsequent to shampooing, conditioning, and/or rinsing the hair, the binding formulation is applied to the hair. Alternately, the hair does not have to be washed or rinsed prior to application of the binding formulation. In this embodiment, the binding formulation is applied to dry hair.

The binding formulations may be used as a daily conditioning treatment for hair.

Typically, the amount of binding formulation applied is sufficient to saturate the hair.

The binding formulation may be applied to the hair as a single application, or application of the binding agent may be repeated one or more times. Typically, the amount of binding formulation applied in each application is sufficient to saturate the hair. The volume of binding formulation applied to the hair in each application may be about 1 to about 100 mL per person depending on their length and volume of hair. In some embodiments, application of the binding agent could be repeated immediately (*e.g.* within about 10 to 15 seconds) or between about one and five minutes, greater than five minutes, between about five and ten minutes, greater than ten minutes, between about ten and twenty (20) minutes after the first application.

### c. Remove the binding formulation from the hair

Preferably, the hair is washed or rinsed subsequent to the application of the binding formulation. The hair may be rinsed and subsequently washed immediately (*e.g.* within 10, 15, 25, 30, 45, 60 seconds (one minute), two minutes, three minutes, four, or five minutes following application) after final application of the binding agent. Alternatively the hair may be rinsed and washed about within about 30 minutes following application, preferably between about 5 minutes and about 20 minutes, more preferably about 10 minutes after the final application of the binding agent to the hair, depending on the hair type.

Alternately, the hair does not have to be washed or rinsed subsequent to application of the binding formulation.

The binding agent covalently binds latent free thiols in the hair. The thiols remain bound for at least one week, preferably for at least one month following application of the binding agent. The thiols may remain bound for longer periods of time, such as for about two months or more following application of the binding agent. The binding reaction is a stable reaction, such that the thiols may remain bound even if subjected to a hair coloring treatment (simultaneous or subsequent to the binding reaction).

### B. Chemical treatment of hair with a reducing agent

In one embodiment, prior to treatment with a binding agent, the hair has been subjected to a reducing agent used for waving (also referred to herein as hair perming or permanent waves), curling, and/or straightening of the hair.

### a. Apply a reducing agent to the hair

The first step in waving, curling, or straightening hair is breaking the cysteine disulfide bonds to form free thiol moieties. The process for breaking the cysteine disulfide bonds is via application of a reducing agent. The process for applying the reducing agent involves following normal perming or hair straightening procedures, that are known to those skilled in the art. For example, to perm a hair, the hair is first washed and set on perm rods of various sizes. Second, a reducing agent, such as thioglycolate reducing solution or lotion, is applied to the hair. The hair is allowed to set for a specified period of time, and then the thioglycolate solution is rinsed from the hair.

The application of hydrogen peroxide in this process is optional. In some processes, such as when treating previously chemically treated hair, hydrogen peroxide is generally not used. In other processes, such as when perming virgin hair, hydrogen peroxide may be added. In these embodiments, hydrogen peroxide is typically added after the reducing agent is rinsed out. Then the hydrogen peroxide is rinsed from the hair prior to adding the binding agent.

### b. Apply the binding agent

Subsequent to the reducing treatment, one or more of the binding agent, or a formulation thereof is applied to the hair. Although the binding agent is typically applied on the same day as treatment with the reducing agent, it may be applied later, such as within 1 to 2 weeks following treatment with the reducing agent.

Typically, the amount of binding formulation applied is sufficient to saturate the hair. The binding agent is generally rinsed and shampooed from the hair after the desired level of hair waving, curling, or straightening is achieved. In some embodiments, the binding agent is rinsed from the hair immediately (*e.g*. within 10, 15, 25, 30, 45, or 60 seconds following application) following the final application of the binding agent. Alternatively the hair may be rinsed and washed about within about 30 minutes following application, preferably between about 5 minutes and about 20 minutes, more preferably about 10 minutes after the final application of the binding agent to the hair, depending on the hair type. The binding agent can be rinsed from the hair within 10, 15, 25, 30, 45, 60 seconds from the hair after application, and still achieve a desired level of hair waving, curling, or straightening.

The binding agent may be applied to the hair as a single application, or application of the binding agent may be repeated one or more times. Typically, the amount of binding formulation applied in each application is sufficient to saturate the hair. In some embodiments, the volume of binding formulation applied to the hair in each application is about 1 to about 10 mL per perm rod. In some embodiments, application of the binding agent could be repeated immediately (*e.g*. within 10 to 15 seconds) or approximately 1, 5, 7.5, 10, 12.5, 15, 17.5, or 20 minutes after the first application. In some embodiments, the second application is about 7 minutes to about 10 minutes after the first application.

The binding agent is rinsed from the hair after its application. The hair may be rinsed and washed immediately (*e.g.* within 10 to 15 seconds following application) after final application of the binding agent. Alternatively the hair may be rinsed and washed about 10 minutes or later after the final application of the binding agent, such as about 15 minutes to about 30 minutes, preferably about 20 minutes after repeated application of the binding agent to the hair.

The binding agent covalently binds the free thiols in the hair. The thiols remain bound for at least one week, two weeks, three weeks, four weeks, one month, two months or more.

The binding agents are generally washed from the individual's hair on the same day as they are applied. In contrast, traditional perms which use only hydrogen peroxide (and do not involve the addition of a cross-linking agent) are generally not washed for at least 48 hours following application (washing the hair prior to 48 hours following a traditional permanent treatment may result in significant loss in the amount of curl in the hair and/or cause damage to the hair).

The compositions described herein improve hair quality, such as appearance (e.g., sheen) and feel, increase dry strength (e.g., tensile strength), and decrease hair breakage when the hair is subjected to subsequent treatments, such as coloring.

In some embodiments, hair breakage decreases by 5, 10, 15, 20, 25, 30, 35, 40, 45, 50, 55, 60, 65, 70, or 75% or higher after treatment with the binding agent compared to untreated hair from the same individual. Hair breakage is a significant problem encountered during coloring and other treatments.

### C. Apply the coloring formulation to the hair

The coloring formulation is generally applied to an individual's hair following normal hair coloring procedures that are known to those skilled in the art. Typically, hair color treatments include two complementary processes: bleaching the hair's natural pigment and/or other artificial pigments present in the hair, and diffusion of dye precursors into the hair, followed by coupling reactions that result in the formation of chromophores within the hair shaft, which are too large to diffuse out of the hair. The hair coloring formulation may be a highlighting formulation, such as formed by mixing bleach powder and developer. More complex colors may contain several precursors and many couplers, and may involve multiple reactions.

The dye precursors may contain several ingredients, each with different functions. The first ingredient is usually an alkalizing agent (usually ammonia and/or an ammonia substitute, such as monoethanolamine [MEA]). The alkalizing agent serves a number of roles in the hair colorant process including swelling the hair fiber to aid in diffusion of the dye precursors. The dye precursors generally include *p-*diamines and *p*-aminophenols. Precursors are oxidized to active intermediates once they have penetrated the hair shaft. Intermediates then react with color couplers to create wash resistant dyes. More specifically, the intermediates, in the presence of an oxidant, couple with another oxidation dye intermediate molecule to form a large fused ring color compound within the hair shaft. The precursor intermediate should penetrate the hair shaft prior to the coupling reaction since the fused ring product is too large to penetrate the hair shaft. Couplers modify the color produced by the oxidation of precursor compounds. The primary difference between demi-permanent and permanent products is the alkalizing agent and the concentration of peroxide. The cuticle does not swell as greatly with demi-permanent dyes, making dye penetration less efficient compared to permanent coloring products.

Several coloring formulations use a reducing agent, such as sodium bisulfate, to break disulfide bonds in the hair, allowing deeper penetration of the hair coloring dyes into the hair. Specifically, the method includes reducing some of the disulfide linkages of the cysteine in the hair shafts to thiol groups while breaking hydrogen bonds. The reducing process changes the chemical and cosmetic characteristics of the hair, which are undesirable.

The hair dyeing process may be followed by a shampoo and conditioning treatment, a neutralizing rinse or an acid balanced shampoo containing in addition to cationic or amphoteric surfactants, cation-active emollients and quarternary polymers. Alternately, the hair dyeing process may be followed by application of the binding formulations described herein, before a shampoo and/or conditioning treatment.

### a. Applying binding formulation

The binding formulation may be applied simultaneously with the hair coloring formulation or subsequently to the application of the hair coloring formulation. For example, the binding formulation may be mixed with the hair coloring treatment and the mixture, containing both the binding formulation the hair coloring treatment, may be applied to the hair.

Alternatively, subsequent to coloring the hair, the binding formulation, or a formulation thereof is applied to the hair. Although the binding agent is typically applied on the same day as the coloring treatment, it may be applied later such as within 1 to 2 weeks following treatment with the reducing agent. Typically, the amount of binding formulation (or a mixture of the binding formulation and the hair coloring formulation) applied is enough to saturate the hair. The binding formulation may be applied to the hair as a single application, or application of the binding agent may be repeated one or more times. Typically, the amount of binding formulation applied in each application is sufficient to saturate the hair. The volume of binding formulation applied to the hair in each application may be about 1 to about 100 mL per person depending on their length and volume of hair. In some embodiments, application of the binding agent could be repeated immediately (e.g. within 10 to 15 seconds) or approximately 1, 5, 7.5, 10, 12.5, 15, 17.5, or 20 minutes after the first application.

The binding agent can be rinsed and shampooed from the hair immediately following application, for example within 10, 15, 25, 30, 45, or 60 seconds, or two, three, four, or five minutes after application. Alternatively, the binding agent may be rinsed from the hair within about 30 minutes following application, preferably between about 5 minutes and about 20 minutes, more preferably about 10 minutes after application of the binding agent to the hair, depending on hair type.

If the binding formulation is combined with the hair coloring treatment and applied as a mixture to the hair, then the mixture remains on the hair as long as needed for the hair coloring treatment. Typically the mixture is applied for approximately 10 minutes. The mixture is removed from the hair in accordance with standard methods for hair coloring treatments, *e.g*., rinse and shampoo, approximately 10 minutes after applying the mixture.

The binding formulation is rinsed from the hair after its application. The hair may be rinsed and subsequently washed immediately (*e.g.* within 10 to 15 seconds following application) after final application of the binding agent. Preferably, the hair is rinsed and/or washed about 10 minutes or later after the final application of the binding agent, such as about 15 minutes to about 30 minutes, optionally about 20 minutes after repeated application of the binding agent to the hair.

The binding agent covalently binds the free thiols in the hair. The thiols remain bound for at least one week, two weeks, three weeks, four weeks, one month, or two months, or more.

The binding agents are generally washed from the individual's hair on the same day as they are applied. In contrast, traditional perms which use only hydrogen peroxide (and do not involve the addition of a cross-linking agent) are generally not washed for at least 48 hours following application (washing the hair prior to 48 hours following a traditional permanent treatment may result in significant loss in the amount of curl in the hair and/or cause damage to the hair).

The compositions described herein improve hair quality, such as appearance (e.g., sheen) and feel, increase dry strength (e.g., tensile strength), and decrease hair breakage when the hair is subjected to subsequent treatments, such as coloring.

In some embodiments, hair breakage decreases by 5, 10, 15, 20, 25, 30, 35, 40, 45, 50, 55, 60, 65, 70, 75, 80, 85,or 90% or higher after treatment with the binding agent compared to untreated hair from the same individual. Hair breakage is a significant problem encountered during coloring and other treatments.

### Examples

### Example 1: Comparison of traditional perm versus perm using bismaleate binding agent

### General

Hair samples were obtained from a human subject and cut in ½ inch wide wefts.

*Reducing agents*: Ammonium thioglycolate (ATG) was obtained from a permanent wave kit manufactured by Zotos. 300 mg of Dithiothreitol in a 10g solution was also used as the reducing agent.

*Binding formulation*: A bismaleate binding agent 2,2'-(ethane-1,2-diylbis(oxy))bis(ethan-1-amine) di-maleate at a concentration of 300 mg in 10 g total solution (water) was used.

### Methods

### Method for perming hair using the binding agents

The hair was washed with clarifying shampoo, towel dried, and then rolled around a perm rod. Ammonium thioglycolate or dithiothreitol was then applied to the hair and left on the hair for 10 minutes to 1 hour. The hair was then rinsed for 30 seconds to 1 minute and then blotted dry with a towel.

The binding formulation was applied to the hair, via a needle nose applicator, drenching the hair. The binding agent was left on the hair for a period of about 7.5 minutes. The hair was drenched for a second time with the binding formulation and left for a second 7.5 minutes, for a total of 15 minutes. The hair was then rinsed with water for about 1-2 minutes then unrolled from the perm rods. After the hair was removed from the perm rods, the hair was shampooed and conditioned with various salon shampoo and conditioner brands, including LiQWd® Hydrating Shampoo and Hydrating Conditioner. The washing and drying steps were repeated 40 times.

A second portion of hair was permed as described above, except, hydrogen peroxide was used instead of the binding formulation.

### Results

Both perms (utilizing the binding formulation or hydrogen peroxide) showed only slight reduction in the overall curl after 40 cycles of washing and drying with the same shampoo and conditioner. However, the appearance and texture of the perm using the binding formulation showed more sheen and less frizz compared to the perm using hydrogen peroxide.

### Example 2: Comparison of hair breakage due to repeated application of traditional perm and the binding formulations.

### Methods

Two hair samples were obtained. Both samples were treated with dithiothreitol or ammonium thioglycolate as described in Example 1. One of the hair samples was subsequently treated with the binding formulation, while the other was neutralized with hydrogen peroxide. The process was completed the same day for the hair treated with the binding formulation. The process was completed in three days with hydrogen peroxide (traditional perm).

The procedure was repeated three times for each hair sample over a 48 hour time period.

### Results

Upon visual inspections, the second hair sample treated with the binding formulation showed little or no signs of breakage. However, the first hair sample treated with hydrogen peroxide showed significant breakage.

### Example 3: Comparison of the extent of damage to hair previously relaxed with a Japanese relaxer

### Methods

Two samples of hair, the first previously straightened with a Japanese relaxer (Yuko), and the second previously straightened with a no lye relaxer (African Pride Miracle Deep Conditioning) were obtained. The samples were treated as described in Examples 1 and 2 using the binding formulation.

Another hair sample, previously straightened with a no lye relaxer (African Pride Miracle Deep Conditioning) was obtained. The sample was treated with a traditional hair straightening perm (Zotos).

### Results

The hair samples treated with the binding formulation showed no noticeable damage. However, the sample treated with a traditional perm showed significant breaking, even during application.

### Example 4: Hair sheen and texture after treatment with binding formulation.

### General

A sample of untreated virgin gray hair was obtained from a human subject.

*Binding formulation:* The bismaleate binding agent in Example 1 (300 mg) was dissolved in water (10 g). The resulting solution was mixed with LiQWD Volumizing Conditioner® in a 1:1 ratio.

### Methods

A section of the virgin gray hair was washed with LiQWD® Hydrating Shampoo and then blotted dry with a towel. The hair was then combed with a wide tooth comb followed by combing with a fine tooth comb for 2 minutes.

After combing, the binding formulation (about 4 mL) was applied to the hair sample by hand and then the sample combed through for approximately 1 minute. The hair sample was left undisturbed for a period of about 10 minutes, after which it was rinsed with water, and then washed with LiQWD® Volumizing Shampoo and Conditioner before being examined.

The hair sample was washed and conditioned for an additional five (5) times with LiQWD® Volumizing Shampoo and Conditioner.

A second section of the virgin gray hair, the control, was treated identically as above, except the binding formulation was not applied to the control hair sample. Thus after the hair was combed, LiQWD Volumizing Conditioner® (without a binding agent) was applied to the hair sample by hand.

### Results:

The hair sample treated with the binding formulation had more shine and felt softer to the touch that the original untreated sample. The treated hair sample gave an overall healthier appearance compared to the control sample.

The shine, texture, and overall appearance remained intact after five shampoo and conditioning treatments.

### Example 5: Hair sheen and texture after treatment with binding formulation.

### General

A sample of untreated virgin blonde hair described as highly porous and difficult to comb through was obtained from a human subject.

*Binding formulation*: The bismaleate binding agent in Example 1 (300 mg) was dissolved in water (10 g). The resulting solution was mixed with LiQWD Enhancing Conditioner® in a 1:1 ratio.

### Methods

A section of the virgin blonde hair was washed with LiQWD® Hydrating Shampoo and then blotted dry with a towel. The hair was then combed with a wide tooth comb followed by combing with a fine tooth comb for 5 minutes.

The binding formulation (about 7 mL) was then applied to the hair sample by hand and the sample combed through for approximately 2 minutes. The hair sample was left undisturbed for a period of about 5 minutes after which the hair was treated again with the binding formulation (about 4 mL). The hair sample was combed through for approximately 10 seconds and left undisturbed for about 5 minutes.

The hair sample was then rinsed with water then washed with LiQWD® Sulfate Free Enhancing Shampoo and Conditioner before examination.

Following initial examination, the sample was washed and conditioned for an additional two (2) times with LiQWD® Sulfate Free Enhancing Shampoo and Conditioner.

A second section of the virgin blonde hair, the control, was treated identically as above, except the binding formulation was not applied to the control hair sample. Thus, after the hair was combed, LiQWD Volumizing Conditioner® (without a binding agent) was applied to the hair sample by hand.

### Results:

The hair sample treated with the binding formulation had more shine and felt softer to the touch than the original untreated sample. The treated hair sample gave an overall healthier appearance compared to the control sample.

The shine, texture, and overall appearance remained intact after two shampoo and conditioning treatments.

### Example 6: Color retention and texture of colored hair treated with the binding formulation.

### General

Three hair samples were obtained from a human subject and cut in ½ inch wide wefts.

*Coloring formulation*: The permanent hair coloring formulation was obtained from a L'Oreal® permanent hair coloring service (L'Oreal® Majirel permanent color #10 with 20 volume peroxide).

*Binding formulatioh*: A bismaleate binding agent, 2,2-(ethane-1,2-diylbis(oxy))bis(ethan-1-amine) di-maleate, at a concentration of 300 mg in 10 g total solution (water) was used.

### Methods

The hair samples were washed with a clarifying shampoo then towel dried. The samples were then colored with the L'Oreal® permanent hair color service, which was left on the hair samples for approximately 35-40 minutes.

The first color treated hair sample ("control") was subsequently rinsed and washed with Liqwd® Hydrating Shampoo and Conditioner five times before being photographed.

The binding formulation was applied to the second and third color treated hair samples via a spray bottle and massaging using the fingers. The binding formulation was left on the second hair sample for a period of about 1 minute and on the third sample for a period of about 10 minutes. The hair samples were subsequently rinsed, and then washed with Liqwd® Hydrating Shampoo and Conditioner five times before being examined.

### Results:

The hair samples treated with the binding formulation showed better color retention, more shine, and less frizz than the control. The hair samples treated with the binding formulation felt smoother to the touch and combined with the lower frizz and added sheen gave an overall healthier appearance over the control.

### Example 7: Comparison of color retention in traditionally permed hair and hair permed using the binding formulations.

### Method

A ½ inch wide weft of hair sample, obtained from a human subject, was washed with clarifying shampoo then towel dried. Ammonium thioglycolate or dithiothreitol was mechanically pulled through the hair with a wide and a fine toothcomb several times then left on the hair for 10 minutes to 1 hour. The hair was then rinsed for 30 seconds to 1 minute with water, and then towel dried.

The binding formulation, described in Example 1, was then applied via a needle nose applicator drenching the hair and leaving it on for 7.5 minutes. This step was repeated, for a total of 15 minutes. The hair was then rinsed for 1-2 minutes, shampooed, and then conditioned with various salon shampoo and conditioner brands, including LiQWd® Hydrating Shampoo and Hydrating Conditioner.

A second sample of hair was straightened, as described above, but using hydrogen peroxide instead of the binding formulation. The hair samples were washed and conditioned repeatedly.

### Comparison of hair color:

After both hair samples were washed five times using LiQWd® Hydrating Shampoo and LiQWd® Hydrating Conditioner, the samples were examined for their color retention.

### Results

The hair sample treated with the binding formulation displayed a color closer in intensity to the hair sample prior to the first washing, compared to the hair treated with hydrogen peroxide.

### Example 8: Comparison of hair treated with highlighting formulation applied simultaneously with binding formulation and hair treated with highlighting formulation alone

The binding formulation in Example 1 contained the bismaleate binding agent at concentrations of 2400 mg in 10 g total solution (water).

Two swatches of human hair were tested. A sample was taken from the same head, 1 inch wide, and split in half. The color was medium brown and had been previously color treated with an unknown professional hair color.

Swatch 1, 1/2 inch wide and 8 inches long, was lightened with traditional highlighting ingredients mixed with a binding formulation. 1oz of Joico Verocolor Veroxide developer-20 volume was mixed with 1oz Joico Verolight powder bleach to form the highlighting formulation. Then 9mL of the binding formulation was added to the highlighting formulation to form a mixture.

The mixture was applied on the Swatch 1hair with an applicator brush as the hair lay on aluminum foil. The foil was then wrapped around the swatch and allowed to process for 35 minutes. The swatch was rinsed and shampooed one time.

Swatch 2, the control, 1/2 inch wide and 8 inches long, was lightened with traditional highlighting ingredients in the absence of a binding formulation. 1oz of Joico Verocolor Veroxide developer-20 volume was mixed with 1oz Joico Verolight powder bleach to form a highlighting formulation with a creamy consistency.

The highlighting formulation was applied on the Swatch 2 hair with an applicator brush as the hair lay on aluminum foil. The foil was then wrapped around the swatch and allowed to process for 35 minutes. The swatch was rinsed and shampooed one time.

### Results

A noticeable difference in hair quality between Swatch 1 and Swatch 2 was observed. Swatch 1 hair was softer, less frizzy, appeared hydrated, with more shine than the control, Swatch 2.

Both swatches were washed and conditioned 5 more times with the same noticeable benefits of Swatch 1 (treated with the mixture of highlighting formulation and binding formulation) compared to the control, Swatch 2 (treated with highlighting formulation, alone).

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific embodiments of the invention described herein. Such equivalents are intended to be encompassed by the following claims.

## Claims

1. A method for treating hair, wherein the hair comprises two or more free thiol groups, comprising:
(a) applying to the hair a formulation comprising a binding agent of Formula I 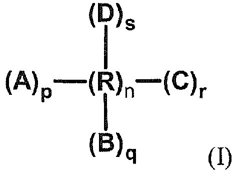 wherein
A, B, C, and D are reactive moieties capable of reacting with the free thiol groups each containing one or more charges, wherein each of the A, B, C, and D reactive moieties independently contain a moiety selected from the group consisting of a vinyl sulfone, an acrylate group, a methacrylate group, a styrene group, an acryl amide group, a methacryl amide group, a maleate group, a fumarate group, and an itaconate group;
(R)ₙ is a linker that contains two or more charges, wherein the charges are opposite to the charges on the reactive moieties, wherein R is a monomer, n = 1-10, and wherein (R)ₙ is not a polymer, and
the sum of the charges is zero, and
wherein the reactive moieties are ionically bound to the linker;
wherein each occurrence of p, q, r, and s is independently an integer from 0 to 25, and wherein the sum of p + q + r + s is equal to or greater than 2, in an effective amount to covalently bind the free thiol groups;
provided that the binding agent of formula I is not a trimaleate salt of
H₂N-(CH₂)₃-N¹(G)-(CH₂)₄-NH₂
wherein G is a substituent that is bound to the secondary amine group of spermidine, chosen from saturated or unsaturated, linear or branched alkyl groups formed from 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene; aryl or aryl-alkyl groups, such as phenyl, naphthyl, benzyl, tolyl, wherein one or more carbon atoms are optionally replaced by fluorine, and wherein said aryl-alkyl groups include saturated or unsaturated, linear or branched alkyl groups formed by 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene; saturated or unsaturated cycloalkyl groups formed by 3 to 8 atoms of carbon that are optionally replaced by saturated or unsaturated, linear or branched alkyl groups formed by 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene.

2. The method of claim 1, wherein the reactive moieties and the thiol groups react to form carbon-sulfur (C-S) covalent bonds.

3. The method of any of claims 1-2, wherein A, B, C, and D are the same.

4. The method of any of claims 1-2, wherein at least one of A, B, C, and D is different than the other reactive moieties.

5. The method of any of claims 1-4, wherein the linker is a polyfunctional molecule, and wherein the linker is optionally independently substituted with one or more substituents including hydrogen, halogen, cyano, alkoxy, alkyl, alkenyl, cycloalkyl, cycloalkenyl, aryl, heterocycloalkyl, heteroaryl, amine, hydroxy, formyl, acyl, carboxylic acid (-COOH), -C(O)R¹, -C(O)OR¹, carboxylate (-COO-), primary amide (*e.g*., -CONH₂), secondary amide (*e.g*., -CONHR¹), -C(O)NR¹R², -NR¹R², -NR¹S(O)₂R², -NR¹C(O)²'-, -S(O)₂R², -SR¹, and - S(O)₂NR¹R², sulfinyl group (*e.g*., -SOR¹), and sulfonyl group (*e.g*., -SOOR¹);
wherein R¹ and R² may each independently be hydrogen, alkyl, alkenyl, alkynyl, cycloalkyl, aryl, heterocycloalkyl and heteroaryl; wherein each of R¹ and R² is optionally independently substituted with one or more substituents selected from the group consisting of halogen, hydroxyl, cyano, nitro, amino, alkylamino, dialkylamino, alkyl optionally substituted with one or more halogen or alkoxy or aryloxy, aryl optionally substituted with one or more halogen or alkoxy or alkyl or trihaloalkyl, heterocycloalkyl optionally substituted with aryl or heteroaryl or =O or alkyl optionally substituted with hydroxyl, cycloalkyl optionally substituted with hydroxyl, heteroaryl optionally substituted with one or more halogen or alkoxy or alkyl or trihaloalkyl, haloalkyl, hydroxyalkyl, carboxy, alkoxy, aryloxy, alkoxycarbonyl, aminocarbonyl, alkylaminocarbonyl, and dialkylaminocarbonyl.

6. The method of any of claims 1-5, wherein the binding agent is: 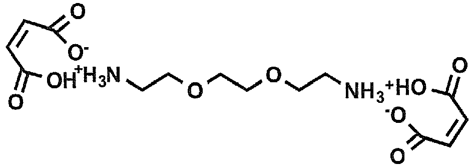 or

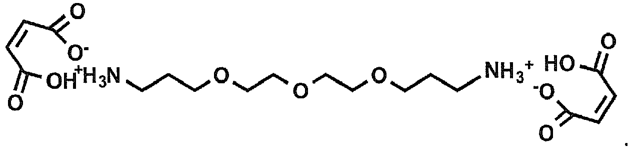

7. The method of any of claims 1-6, wherein the A, B, C and D reactive moieties independently contain a moiety selected from the group consisting of vinyl sulfone, a styrene group, an acryl amide group, a methacryl amide group, a maleate group and a fumarate group.

8. The method of any of claims 1-7, wherein the molecular weight of the binding agent is less than about 500 Daltons.

9. The method of any of claims 1-8, wherein the formulation further comprises one or more cosmetically acceptable excipients,
wherein the one or more excipients are selected from the group consisting of water, surfactants, vitamins, natural extracts, preservatives, chelating agents, perfumes, preservatives, antioxidants, chelating agents, hair coloring agents, proteins, amino acids, humectants, fragrances, emollients, penetrants, thickeners, viscosity modifiers, hair fixatives, film formers, emulsifiers, opacifying agents, propellants, liquid vehicles, carriers, salts, pH adjusting agents, neutralizing agents, buffers, hair conditioning agents, anti-static agents, anti-frizz agents, anti-dandruff agents, and combinations thereof.

10. The method of claim 9, wherein the binding agent is present in an amount ranging from 0.01 wt% to 50 wt% of the formulation.

11. The method of any of claims 9-10, wherein the binding agent is about 3 wt% of the formulation.

12. The method of any of claims 9-11, wherein the excipient is present in an amount ranging from 10 wt% to 90 wt% of the formulation.

13. The method of any of claims 1-12, wherein the formulation is in the form of a gel, cream, lotion, shampoo, or conditioner.

14. The method of any of claims 1-13, wherein step (a) is repeated one or more times.

15. The method of any of claims 1-14, wherein step (a) is repeated 1 minutes to 20 minutes after the first application of the formulation.

16. The method of any of claims 1-15, further comprising,
(b) rinsing, shampooing, and/or conditioning the hair, wherein step (b) occurs subsequent to step (a).

17. The method of claim 16, wherein step (b) is performed within 10 seconds to 30 minutes after step (a).

18. The method of any of claims 1-17, further comprising the step of:
applying a first formulation comprising a reducing agent capable of reducing the disulfide bonds in the hair to produce free thiol groups,
wherein the step is performed prior to applying the formulation comprising the binding agent.

19. The method of claim 18, wherein the reducing agent is selected from the group consisting of thioglycolic acid and its derivative salts and esters, thiolactic acid and its derivative salts and esters, cysteine and its derivatives, cysteamine and its derivatives, inorganic sulfites, sodium metabisulfite, other inorganic bisulfites, dithiothreitol, dithioerythritol, organic phosphines, and Japanese relaxers.

20. The method of any of claims 1-19, wherein the thiols remain bound for at least one week.

21. The method of any of claims 1-20, wherein the molecular weight of the binding agent is less than 500 Daltons.

22. A formulation consisting of a binding agent, an aqueous solvent, and one or more preservatives, stabilizers, or combinations thereof,
wherein the binding agent is represented by Formula I: 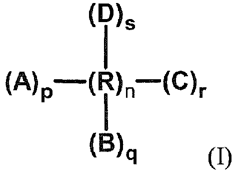 wherein
A, B, C, and D are reactive moieties capable of reacting with the free thiol groups each containing one or more charges, wherein each of the A, B, C, and D reactive moieties independently contain a moiety selected from the group consisting of a vinyl sulfone, an acrylate group, a methacrylate group, a styrene group, an acryl amide group, a methacryl amide group, a maleate group, a fumarate group, and an itaconate group;
(R)ₙ is a linker that contains two or more charges, wherein the charges are opposite to the charges on the reactive moieties, wherein R is a monomer, n = 1-10, and wherein (R)ₙ is not a polymer, and the sum of the charges is zero, and
wherein the reactive moieties are ionically bound to the linker;
wherein each occurrence of p, q, r, and s is independently an integer from 0 to 25, and wherein the sum of p + q + r + s is equal to or greater than 2.

23. The formulation of claim 22, wherein the binding agent is: 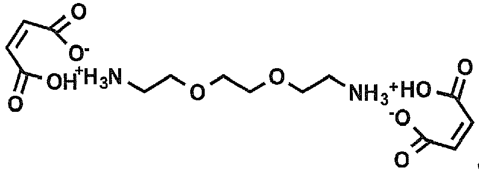 or

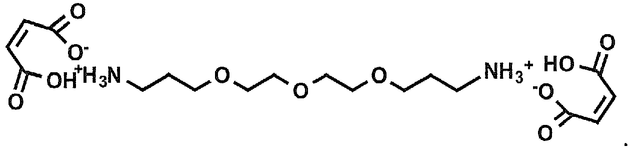

24. A method for coloring hair comprising:
(a) applying a first formulation comprising a hair coloring agent and a reducing agent capable of reducing the disulfide bonds in the hair to produce free thiol groups,
(b) applying to the hair a second formulation comprising a binding agent of Formula I 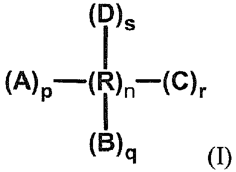 wherein
A, B, C, and D are reactive moieties capable of reacting with the free thiol groups each containing one or more charges, wherein each of the A, B, C, and D reactive moieties independently contain a moiety selected from the group consisting of a vinyl sulfone, an acrylate group, a methacrylate group, a styrene group, an acryl amide group, a methacryl amide group, a maleate group, a fumarate group, and an itaconate group;
(R)ₙ is a linker that contains two or more charges, wherein the charges are opposite to the charges on the reactive moieties, wherein R is a monomer, n = 1-10, and wherein (R)ₙ is not a polymer, and the sum of the charges is zero, and
wherein the reactive moieties are ionically bound to the linker;
wherein each occurrence of p, q, r, and s is independently an integer from 0 to 25, and wherein the sum of p + q + r + s is equal to or greater than 2,
in an effective amount to covalently bind the free thiol groups.

25. A method for highlighting hair comprising:
(a) applying a first formulation comprising bleach to lighten hair and produce free thiol groups,
(b) applying to the hair a second formulation comprising a binding agent of Formula I 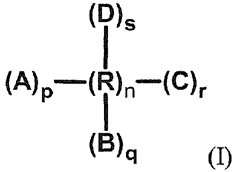 wherein
A, B, C, and D are reactive moieties capable of reacting with the free thiol groups each containing one or more charges, wherein each of the A, B, C, and D reactive moieties independently contain a moiety selected from the group consisting of a vinyl sulfone, an acrylate group, a methacrylate group, a styrene group, an acryl amide group, a methacryl amide group, a maleate group, a fumarate group, and an itaconate group;
(R)ₙ is a linker that contains two or more charges, wherein the charges are opposite to the charges on the reactive moieties, wherein R is a monomer, n = 1-10, and wherein (R)ₙ is not a polymer, and the sum of the charges is zero, and
wherein the reactive moieties are ionically bound to the linker;
wherein each occurrence of p, q, r, and s is independently an integer from 0 to 25, and wherein the sum of p + q + r + s is equal to or greater than 2,
in an effective amount to covalently bind the free thiol groups.

26. The method of claim 24 or 25, wherein steps (a) and (b) are performed simultaneously.

27. The method of claim 24 or 25, wherein steps (a) and (b) are performed sequentially, with step (a) performed prior to step (b).

28. The method of claim 26, wherein prior to step (a), the first formulation and the second formulation are mixed together.

29. The method of any of claims 24-28, wherein the reactive moieties and the thiol groups react to form carbon-sulfur (C-S) covalent bonds.

30. The method of any of claims 24-29, wherein A, B, C, and D are the same.

31. The method of any of claims 24-29, wherein at least one of A, B, C, and D is different than the other reactive moieties.

32. The method of any of claims 24-31, wherein the linker is a polyfunctional molecule,
wherein the linker is optionally independently substituted with one or more substituents including hydrogen, halogen, cyano, alkoxy, alkyl, alkenyl, cycloalkyl, cycloalkenyl, aryl, heterocycloalkyl, heteroaryl, amine, hydroxy, formyl, acyl, carboxylic acid (-COOH), -C(O)R¹, -C(O)OR¹, carboxylate (-COO⁻), primary amide (e.g., -CONH₂), secondary amide (*e.g*., -CONHR¹), -C(O)NR¹R², -NR¹R², -NR¹S(O)₂R², -NR¹C(O)R², - S(O)₂R², -SR¹, and -S(O)₂NR¹R², sulfinyl group (*e.g.,* -SOR¹), and sulfonyl group (*e.g.,* - SOORI¹);
wherein R¹ and R² may each independently be hydrogen, alkyl, alkenyl, alkynyl, cycloalkyl, aryl, heterocycloalkyl and heteroaryl; wherein each of R¹ and R² is optionally independently substituted with one or more substituents selected from the group consisting of halogen, hydroxyl, cyano, nitro, amino, alkylamino, dialkylamino, alkyl optionally substituted with one or more halogen or alkoxy or aryloxy, aryl optionally substituted with one or more halogen or alkoxy or alkyl or trihaloalkyl, heterocycloalkyl optionally substituted with aryl or heteroaryl or =O or alkyl optionally substituted with hydroxyl, cycloalkyl optionally substituted with hydroxyl, heteroaryl optionally substituted with one or more halogen or alkoxy or alkyl or trihaloalkyl, haloalkyl, hydroxyalkyl, carboxy, alkoxy, aryloxy, alkoxycarbonyl, aminocarbonyl, alkylaminocarbonyl, and dialkylaminocarbonyl.

33. The method of any of claims 24-32, wherein the binding agent is: 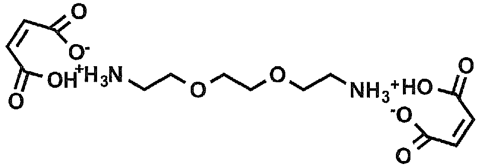 or

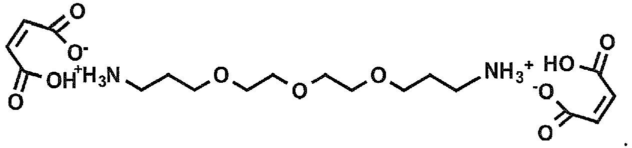

34. The method of any of claims 24-33, wherein the second formulation further comprises one or more cosmetically acceptable excipients, and
wherein the one or more excipients are selected from the group consisting of water, surfactants, vitamins, natural extracts, preservatives, chelating agents, perfumes, preservatives, antioxidants, proteins, amino acids, humectants, fragrances, emollients, penetrants, thickeners, viscosity modifiers, hair fixatives, film formers, emulsifiers, opacifying agents, propellants, liquid vehicles, carriers, salts, pH adjusting agents, neutralizing agents, buffers, hair conditioning agents, anti-static agents, anti-frizz agents, anti-dandruff agents, and combinations thereof.

35. The method of claim 34, wherein the binding agent is present in an amount ranging from 0.01 wt% to 50 wt% of the second formulation, preferably from 0.01 wt% to 10 wt% of the second formulation.

36. The method of any of claims 33-35, wherein the binding agent is present in an amount ranging from 2.5 to 3 wt% of the second formulation.

37. The method of any of claims 33-36, wherein the excipient is present in an amount ranging from 10 wt% to 99.99 wt% of the second formulation, preferably from 50 wt% to 90 wt% of the second formulation.

38. The method of any of claims 24-37, wherein the second formulation is in the form of a gel, cream, lotion, shampoo, or conditioner.

39. The method of any of claims 24-38, wherein step (b) is repeated one or more times.

40. The method of any of claims 24-39, further comprising,
(c) rinsing, shampooing, and/or conditioning the hair, wherein step (c) occurs subsequent to step (b).

41. The method of claim 40, wherein step (c) is performed within 10 seconds to 30 minutes, preferably between 1 minute and 20 minutes, more preferably about 10 minutes after step (b).

42. The method of any of claims 24-41, wherein the coloring agent is selected from the group consisting of highlighting agents, permanent coloring agents, demi-permanent coloring agents, and semi-permanent coloring agents.

43. A kit comprising:
(a) a first formulation comprising a reducing agent capable of reducing disulfide bonds in hair to produce free thiol groups, and
(b) a second formulation comprising a binding agent in an effective amount to covalently bind free thiol groups in hair,
wherein the binding agent is represented by Formula I: 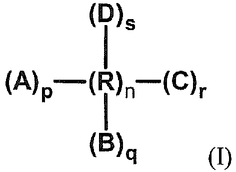 wherein
A, B, C, and D are reactive moieties capable of reacting with the free thiol groups each containing one or more charges, wherein each of the A, B, C, and D reactive moieties independently contain a moiety selected from the group consisting of a vinyl sulfone, an acrylate group, a methacrylate group, a styrene group, an acryl amide group, a methacryl amide group, a maleate group, a fumarate group, and an itaconate group;
(R)ₙ is a linker that contains two or more charges, wherein the charges are opposite to the charges on the reactive moieties, wherein R is a monomer, n = 1-10, and wherein (R)ₙ is not a polymer, and the sum of the charges is zero, and
wherein the reactive moieties are ionically bound to the linker;
wherein each occurrence of p, q, r, and s is independently an integer from 0 to 25, and wherein the sum of p + q + r + s is equal to or greater than 2.

44. The kit of claim 43, further comprising a shampoo, a conditioner, instructions for use, a developer, mixing container, an odor eliminator, gloves, or a combination thereof.

45. The kit of any of claims 43-44, wherein the first formulation is a bleaching, straightening, waving, curling, or coloring formulation.

46. The kit of any of claims 43-45, wherein A, B, C, and D are the same.

47. The kit of any of claims 43-46, wherein at least one of A, B, C, and D is different than the other reactive moieties.

48. The kit of any of claims 43-47, wherein the linker is a polyfunctional molecule,
wherein the linker is optionally independently substituted with one or more substituents selected from the group consisting of hydrogen, halogen, cyano, alkoxy, alkyl, alkenyl, cycloalkyl, cycloalkenyl, aryl, heterocycloalkyl, heteroaryl, amine, hydroxy, formyl, acyl, carboxylic acid (-COOH), -C(O)R¹, -C(O)OR¹, carboxylate (-COO⁻), primary amide (*e.g.,* -CONH₂), secondary amide (*e.g.,* -CONHR¹), -C(O)NR¹R², -NR¹R², -NR¹S(O)₂R², -NR¹C(O)R², -S(O)₂R² , -SR¹, and -S(O)₂NR¹R², sulfinyl group (*e.g.,* -SOR¹), and sulfonyl group (*e.g.,* -SOOR¹);
wherein R¹ and R² may each independently be hydrogen, alkyl, alkenyl, alkynyl, cycloalkyl, aryl, heterocycloalkyl and heteroaryl; wherein each of R¹ and R² is optionally independently substituted with one or more substituents selected from the group consisting of halogen, hydroxyl, cyano, nitro, amino, alkylamino, dialkylamino, alkyl optionally substituted with one or more halogen or alkoxy or aryloxy, aryl optionally substituted with one or more halogen or alkoxy or alkyl or trihaloalkyl, heterocycloalkyl optionally substituted with aryl or heteroaryl or =O or alkyl optionally substituted with hydroxyl, cycloalkyl optionally substituted with hydroxyl, heteroaryl optionally substituted with one or more halogen or alkoxy or alkyl or trihaloalkyl, haloalkyl, hydroxyalkyl, carboxy, alkoxy, aryloxy, alkoxycarbonyl, aminocarbonyl, alkylaminocarbonyl, and dialkylaminocarbonyl.

49. The kit of any of claims 43-48, wherein the reactive moieties and the thiol groups react to form carbon-sulfur (C-S) covalent bonds.

50. The kit of any of one of claims 43-49, wherein the binding agent is: 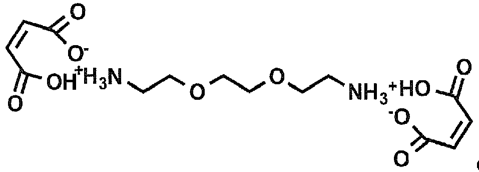 or

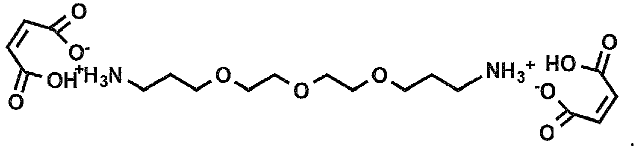

51. The kit of any of claims 43-50, wherein the second formulation further comprises one or more cosmetically acceptable excipients selected from the group consisting of water, surfactants, vitamins, natural extracts, preservatives, chelating agents, perfumes, preservatives, antioxidants, chelating agents, hair coloring agents, proteins, amino acids, humectants, fragrances, emollients, penetrants, thickeners, viscosity modifiers, hair fixatives, film formers, emulsifiers, opacifying agents, propellants, liquid vehicles, carriers, salts, pH adjusting agents, neutralizing agents, buffers, hair conditioning agents, anti-static agents, anti-frizz agents, anti-dandruff agents, and combinations thereof.

52. The kit of any of claims 43-51, wherein the binding agent is present in an amount ranging from 0.01 wt% to 50 wt% of the second formulation.

53. The kit of any of claims 43-50, wherein the excipient is present in an amount ranging from 10 wt% to 90 wt% of the second formulation.

54. The kit of any of claims 43-53, wherein the first and second formulations are pre-mixed.

55. The kit of any of claims 43-54, wherein the second formulation is pre-mixed with a shampoo or conditioner.

56. The kit of any of claims 43-55, wherein the second formulation is in the form of a liquid, lotion, milk, mousse, spray, gel, cream, shampoo, or conditioner.

57. The kit of any of claims 43-56, wherein the second formulation is provided as two or more separate ingredients.

58. The kit of any of claims 43-57, wherein the binding agent is provided as a dry powder in a sealed package and the pharmaceutically acceptable excipient provided in a vial or other container.

59. The kit of any of claims 43-58, wherein:
(1) the first formulation is a bleaching formulation; and
(2) the binding agent of the second formulation is

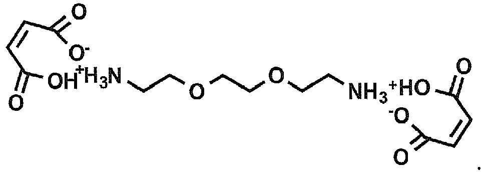

60. The kit of claim 59, wherein the bleaching formulation further comprises a developer.

61. The kit of any of claims 59-60, further comprising a shampoo, a conditioner, instructions for use, a developer, mixing container, an odor eliminator, or a combination thereof.

62. A shampoo or conditioner comprising an effective amount of a binding agent, wherein the binding agent is represented by Formula I: 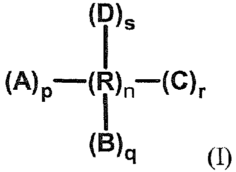 wherein
A, B, C, and D are reactive moieties capable of reacting with the free thiol groups each containing one or more charges, wherein each of the A, B, C, and D reactive moieties independently contain a moiety selected from the group consisting of a vinyl sulfone, an acrylate group, a methacrylate group, a styrene group, an acryl amide group, a methacryl amide group, a maleate group, a fumarate group, and an itaconate group;
(R)ₙ is a linker that contains two or more charges, wherein the charges are opposite to the charges on the reactive moieties, wherein R is a monomer, n = 1-10, and wherein (R)ₙ is not a polymer, and the sum of the charges is zero, and
wherein the reactive moieties are ionically bound to the linker;
wherein each occurrence of p, q, r, and s is independently an integer from 0 to 25, and wherein the sum of p + q + r + s is equal to or greater than 2;
provided that the binding agent of formula I is not a trimaleate salt of
H₂N-(CH₂)₃-N¹(G)-(CH₂)₄-NH₂
wherein G is a substituent that is bound to the secondary amine group of spermidine, chosen from saturated or unsaturated, linear or branched alkyl groups formed from 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene; aryl or aryl-alkyl groups, such as phenyl, naphthyl, benzyl, tolyl, wherein one or more carbon atoms are optionally replaced by fluorine, and wherein said aryl-alkyl groups include saturated or unsaturated, linear or branched alkyl groups formed by 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene; saturated or unsaturated cycloalkyl groups formed by 3 to 8 atoms of carbon that are optionally replaced by saturated or unsaturated, linear or branched alkyl groups formed by 1 to 6 atoms of carbon, wherein one or more carbon atoms are optionally replaced by fluorine, namely methyl, ethyl, trifluoromethyl, trifluoroethyl, propyl, isopropyl, butyl, isobutyl, pentyl, hexyl, ethylene, vinyl, propylene, butylene.

63. The shampoo or conditioner of claim 62, wherein the reactive moieties and the thiol groups react to form carbon-sulfur (C-S) covalent bonds.

64. The shampoo or conditioner of any of claims 62-63, wherein A, B, C, and D are the same.

65. The shampoo or conditioner of any of claims 62-63, wherein at least one of A, B, C, and D is different than the other reactive moieties.

66. The shampoo or conditioner of any of claims 62-65, wherein the linker is a polyfunctional molecule,
wherein the linker is optionally independently substituted with one or more substituents selected from the group consisting of hydrogen, halogen, cyano, alkoxy, alkyl, alkenyl, cycloalkyl, cycloalkenyl, aryl, heterocycloalkyl, heteroaryl, amine, hydroxy, formyl, acyl, carboxylic acid (-COOH), -C(O)R¹, -C(O)OR¹, carboxylate (-COO⁻), primary amide (e.g., -CONH₂), secondary amide (e.g., -CONHR¹), -C(O)NR¹R², -NR¹R², -NR¹S(O)₂R², -NR¹C(O)R², -S(O)₂R² , -SR¹, and -S(O)₂NR¹R² sulfinyl group (e.g., -SOR¹¹), and sulfonyl group (e.g., -SOOR¹);
wherein R¹ and R² may each independently be hydrogen, alkyl, alkenyl, alkynyl, cycloalkyl, aryl, heterocycloalkyl and heteroaryl;
wherein each of R¹ and R² is optionally independently substituted with one or more substituents selected from the group consisting of halogen, hydroxyl, cyano, nitro, amino, alkylamino, dialkylamino, alkyl optionally substituted with one or more halogen or alkoxy or aryloxy, aryl optionally substituted with one or more halogen or alkoxy or alkyl or trihaloalkyl, heterocycloalkyl optionally substituted with aryl or heteroaryl or =O or alkyl optionally substituted with hydroxyl, cycloalkyl optionally substituted with hydroxyl, heteroaryl optionally substituted with one or more halogen or alkoxy or alkyl or trihaloalkyl, haloalkyl, hydroxyalkyl, carboxy, alkoxy, aryloxy, alkoxycarbonyl, aminocarbonyl, alkylaminocarbonyl, and dialkylaminocarbonyl.

67. The shampoo or conditioner of any of claims 62-66, wherein the binding agent is: 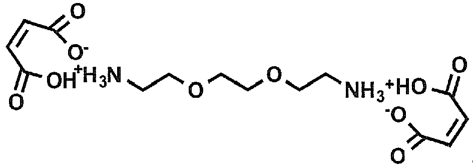 or

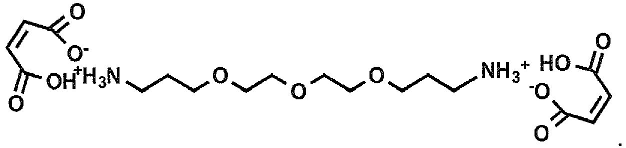

68. The shampoo or conditioner of any of claims 62-67, further comprising one or more cosmetically acceptable excipients, wherein the one or more excipients are selected from the group consisting of water, surfactants, vitamins, natural extracts, preservatives, chelating agents, perfumes, preservatives, antioxidants, chelating agents, hair coloring agents, proteins, amino acids, humectants, fragrances, emollients, penetrants, thickeners, viscosity modifiers, hair fixatives, film formers, emulsifiers, opacifying agents, propellants, liquid vehicles, carriers, salts, pH adjusting agents, neutralizing agents, buffers, hair conditioning agents, anti-static agents, anti-frizz agents, anti-dandruff agents, and combinations thereof.

69. The shampoo or conditioner of claim 68, wherein the binding agent is present in an amount ranging from 0.01 wt% to 50 wt% of the formulation.

70. The shampoo or conditioner of any of claims 68-69, wherein the binding agent is present in an amount ranging from 2.5 to 3 wt% of the formulation.

## Patentansprüche

1. Verfahren zum Behandeln von Haar, wobei das Haar zwei oder mehr freie Thiolgruppen umfasst, Folgendes umfassend:
(a) Aufbringen - auf das Haar - einer Formulierung, die ein Bindemittel der Formel I umfasst 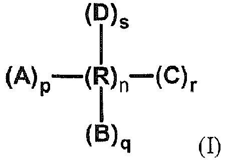 wobei
A, B, C und D reaktionsfähige Komponenten sind, die in der Lage sind, mit den freien Thiolgruppen zu reagieren, und jeweils eine oder mehrere Ladungen enthalten, wobei jede der reaktionsfähigen Komponenten A, B, C und D unabhängig eine Komponente enthält, die ausgewählt ist aus der Gruppe bestehend aus einem Vinylsulfon, einer Acrylatgruppe, einer Methacrylatgruppe, einer Styrolgruppe, einer Acrylamidgruppe, einer Methacrylamidgruppe, einer Maleatgruppe, einer Fumaratgruppe und einer Itaconatgruppe;
R(ₙ) ein Bindeglied ist, das zwei oder mehr Ladungen enthält, wobei die Ladungen den Ladungen an den reaktionsfähigen Komponenten entgegengesetzt sind, wobei R ein Monomer ist, n = 1-10, und wobei (R)ₙ kein Polymer ist und
die Summe der Ladungen Null ist, und
wobei die reaktionsfähigen Komponenten ionisch an das Bindeglied gebunden sind;
wobei jedes Vorkommen von p, q, r und s unabhängig eine ganze Zahl von 0 bis 25 ist und wobei die Summe von p + q + r + s gleich oder größer als 2 ist, in einer wirksamen Menge, um die freien Thiolgruppen kovalent zu binden;
mit der Maßgabe, dass das Bindemittel der Formel I kein Trimaleatsalz von H₂N-(CH₂)₃-N¹(G)-(CH₂)₄-NH₂ ist,
wobei G ein Substituent ist, der an die sekundäre Amingruppe von Spermidin gebunden ist, ausgewählt aus gesättigten oder ungesättigten, linearen oder verzweigten Alkylgruppen, die aus 1 bis 6 Kohlenstoffatomen ausgebildet sind, wobei ein oder mehrere Kohlenstoffatome optional durch Fluor ersetzt ist/sind, konkret Methyl, Ethyl, Trifluormethyl, Trifluorethyl, Propyl, Isopropyl, Butyl, Isobutyl, Pentyl, Hexyl, Ethylen, Vinyl, Propylen, Butylen; Aryl- oder Aryl-Alkyl-Gruppen, wie etwa Phenyl, Naphthyl, Benzyl, Tolyl, wobei ein oder mehrere Kohlenstoffatome optional durch Fluor ersetzt ist/sind und wobei die Aryl-Alkyl-Gruppen gesättigte oder ungesättigte, lineare oder verzweigte Alkylgruppen, die durch 1 bis 6 Kohlenstoffatome ausgebildet sind, umfassen, wobei ein oder mehrere Kohlenstoffatome optional durch Fluor ersetzt ist/sind, konkret Methyl, Ethyl, Trifluormethyl, Trifluorethyl, Propyl, Isopropyl, Butyl, Isobutyl, Pentyl, Hexyl, Ethylen, Vinyl, Propylen, Butylen; gesättigte oder ungesättigte Cycloalkylgruppen, die durch 3 bis 8 Kohlenstoffatome ausgebildet sind, die optional ersetzt sind durch gesättigte oder ungesättigte, lineare oder verzweigte Alkylgruppen, die durch 1 bis 6 Kohlenstoffatome ausgebildet sind, wobei ein oder mehrere Kohlenstoffatome optional durch Fluor ersetzt ist/sind, konkret Methyl, Ethyl, Trifluormethyl, Trifluorethyl, Propyl, Isopropyl, Butyl, Isobutyl, Pentyl, Hexyl, Ethylen, Vinyl, Propylen, Butylen.

2. Verfahren nach Anspruch 1, wobei die reaktionsfähigen Komponenten und die Thiolgruppen reagieren, um kovalente Kohlenstoff-Schwefel(C-S)-Bindungen auszubilden.

3. Verfahren nach einem der Ansprüche 1-2, wobei A, B, C und D gleich sind.

4. Verfahren nach einem der Ansprüche 1-2, wobei wenigstens eines von A, B, C und D anders ist als die anderen reaktionsfähigen Komponenten.

5. Verfahren nach einem der Ansprüche 1-4, wobei das Bindeglied ein polyfunktionales Molekül ist und wobei das Bindeglied optional unabhängig substituiert ist mit einem oder mehreren Substituenten, umfassend Wasserstoff, Halogen, Cyano, Alkoxy, Alkyl, Alkenyl, Cycloalkyl, Cycloalkenyl, Aryl, Heterocycloalkyl, Heteroaryl, Amin, Hydroxy, Formyl, Acyl, Carbonsäure (-COOH), -C(O)R¹, -C(O)OR¹, Carboxylat (-COO-), primäres Amid (z. B. -CONH₂), sekundäres Amid (z. B. -CONHR¹), -C(O)NR¹R², -NR¹R², -NR¹S(O)₂R², -NR¹C(O)R², -S(O)₂R², -SR¹ und -S(O)₂NR¹R², eine Sulfinylgruppe (z. B. -SOR¹) und eine Sulfonylgruppe (z. B. -SOOR¹);
wobei R¹ und R² jeweils unabhängig Wasserstoff, Alkyl, Alkenyl, Alkinyl, Cycloalkyl, Aryl, Heterocycloalkyl und Heteroaryl sein können; wobei jedes von R¹ und R² optional unabhängig substituiert ist mit einem oder mehreren Substituenten, ausgewählt aus der Gruppe bestehend aus Halogen, Hydroxyl, Cyano, Nitro, Amino, Alkylamino, Dialkylamino, Alkyl, optional substituiert mit einem oder mehreren Halogen oder Alkoxy oder Aryloxy, Aryl, optional substituiert mit einem oder mehreren Halogen oder Alkoxy oder Alkyl oder Trihaloalkyl, Heterocycloalkyl, optional substituiert mit Aryl oder Heteroaryl oder =O oder Alkyl, optional substituiert mit Hydroxyl, Cycloalkyl, optional substituiert mit Hydroxyl, Heteroaryl, optional substituiert mit einem oder mehreren Halogen oder Alkoxy oder Alkyl oder Trihaloalkyl, Haloalkyl, Hydroxyalkyl, Carboxy, Alkoxy, Aryloxy, Alkoxycarbonyl, Aminocarbonyl, Alkylaminocarbonyl und Dialkylaminocarbonyl.

6. Verfahren nach einem der Ansprüche 1-5, wobei das Bindemittel Folgendes ist: 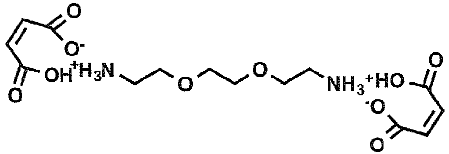 oder

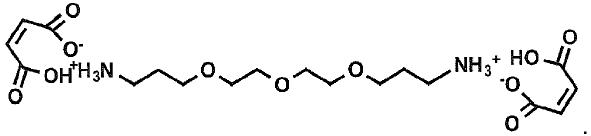

7. Verfahren nach einem der Ansprüche 1-6, wobei die reaktionsfähigen Komponenten A, B, C und D unabhängig eine Komponente enthalten, die ausgewählt ist aus der Gruppe bestehend aus Vinylsulfon, einer Styrolgruppe, einer Acrylamidgruppe, einer Methacrylamidgruppe, einer Maleatgruppe und einer Fumaratgruppe.

8. Verfahren nach einem der Ansprüche 1-7, wobei das Molekulargewicht des Bindemittels weniger als etwa 500 Dalton beträgt.

9. Verfahren nach einem der Ansprüche 1-8, wobei die Formulierung ferner einen oder mehrere kosmetisch annehmbare Hilfsstoffe umfasst,
wobei der eine oder die mehreren Hilfsstoffe ausgewählt ist/sind aus der Gruppe bestehend aus Wasser, oberflächenaktiven Mitteln, Vitaminen, natürlichen Extrakten, Konservierungsmitteln, Chelatbildnern, Parfümen, Konservierungsmitteln, Antioxidationsmitteln, Chelatbildnern, Haarfärbemitteln, Proteinen, Aminosäuren, Feuchthaltemitteln, Duftstoffen, Weichmachern, Eindringmitteln, Verdickungsmitteln, Viskositätsmodifizierern, Haarfixiermitteln, Filmbildnern, Emulgatoren, Trübungsmitteln, Treibmitteln, Trägerflüssigkeiten, Trägern, Salzen, pH-Reglern, Neutralisierungsmitteln, Puffern, Haarkonditionierungsmitteln, Antistatika, Antikräuselmitteln, Antischuppenmitteln und Kombinationen derselben.

10. Verfahren nach Anspruch 9, wobei das Bindemittel in einer Menge im Bereich von 0,01 Gew.-% bis 50 Gew.-% der Formulierung vorhanden ist.

11. Verfahren nach einem der Ansprüche 9-10, wobei das Bindemittel etwa 3 Gew.-% der Formulierung ausmacht.

12. Verfahren nach einem der Ansprüche 9-11, wobei der Hilfsstoff in einer Menge im Bereich von 10 Gew.-% bis 90 Gew.-% der Formulierung vorhanden ist.

13. Verfahren nach einem der Ansprüche 1-12, wobei die Formulierung in Form eines Gels, einer Creme, einer Lotion, eines Shampoos oder einer Pflegespülung vorliegt.

14. Verfahren nach einem der Ansprüche 1-13, wobei Schritt (a) ein oder mehrere Male wiederholt wird.

15. Verfahren nach einem der Ansprüche 1-14, wobei Schritt (a) 1 Minute bis 20 Minuten nach dem ersten Aufbringen der Formulierung wiederholt wird.

16. Verfahren nach einem der Ansprüche 1-15, ferner Folgendes umfassend:
(b) Ausspülen, Shampoonieren und/oder Pflegespülen des Haars, wobei Schritt (b) nach Schritt (a) erfolgt.

17. Verfahren nach Anspruch 16, wobei Schritt (b) innerhalb von 10 Sekunden bis 30 Minuten nach Schritt (a) durchgeführt wird.

18. Verfahren nach einem der Ansprüche 1-17, das ferner folgenden Schritt umfasst:
Aufbringen einer ersten Formulierung, die ein Reduktionsmittel umfasst, das in der Lage ist, die Disulfidbindungen in dem Haar zu reduzieren, um freie Thiolgruppen zu erzeugen,
wobei der Schritt vor dem Aufbringen der das Bindemittel umfassenden Formulierung durchgeführt wird.

19. Verfahren nach Anspruch 18, wobei das Reduktionsmittel ausgewählt ist aus der Gruppe bestehend aus Thioglykolsäure und ihren Derivatsalzen und -estern, Thiomilchsäure und ihren Derivatsalzen und -estern, Cystein und seinen Derivaten, Cysteamin und seinen Derivaten, anorganischen Sulfiten, Natriummetabisulfit, anderen anorganischen Bisulfiten, Dithiothreitol, Dithioerythritol, organischen Phosphinen und japanischen Glättungsmitteln.

20. Verfahren nach einem der Ansprüche 1-19, wobei die Thiole für wenigstens eine Woche gebunden bleiben.

21. Verfahren nach einem der Ansprüche 1-20, wobei das Molekulargewicht des Bindemittels weniger als 500 Dalton beträgt.

22. Formulierung, bestehend aus einem Bindemittel, einem wässrigen Lösungsmittel und einem oder mehreren Konservierungsmitteln, Stabilisatoren oder Kombinationen derselben,
wobei das Bindemittel durch die Formel I dargestellt wird: 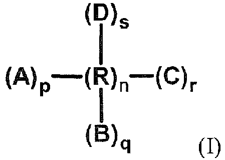 wobei
A, B, C und D reaktionsfähige Komponenten sind, die in der Lage sind, mit den freien Thiolgruppen zu reagieren, und jeweils eine oder mehrere Ladungen enthalten, wobei jede der reaktionsfähigen Komponenten A, B, C und D unabhängig eine Komponente enthält, die ausgewählt ist aus der Gruppe bestehend aus einem Vinylsulfon, einer Acrylatgruppe, einer Methacrylatgruppe, einer Styrolgruppe, einer Acrylamidgruppe, einer Methacrylamidgruppe, einer Maleatgruppe, einer Fumaratgruppe und einer Itaconatgruppe;
R(ₙ) ein Bindeglied ist, das zwei oder mehr Ladungen enthält, wobei die Ladungen den Ladungen an den reaktionsfähigen Komponenten entgegengesetzt sind, wobei R ein Monomer ist, n = 1-10 und wobei (R)ₙ kein Polymer ist und die Summe der Ladungen Null ist, und
wobei die reaktionsfähigen Komponenten ionisch an das Bindeglied gebunden sind;
wobei jedes Vorkommen von p, q, r und s unabhängig eine ganze Zahl von 0 bis 25 ist und wobei die Summe von p + q + r + s gleich oder größer als 2 ist.

23. Formulierung nach Anspruch 22, wobei das Bindemittel Folgendes ist: 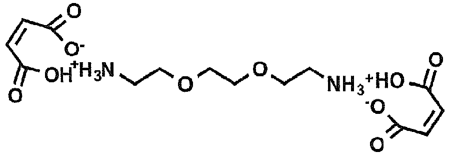 oder

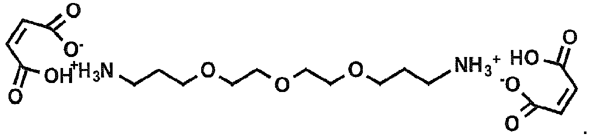

24. Verfahren zum Färben von Haar, Folgendes umfassend: (a) Aufbringen einer ersten Formulierung, umfassend ein Haarfärbemittel und ein Reduktionsmittel, das in der Lage ist, die Disulfidbindungen in dem Haar zu reduzieren, um freie Thiolgruppen zu erzeugen,
(b) Aufbringen - auf das Haar - einer zweiten Formulierung, die ein Bindemittel der Formel I umfasst 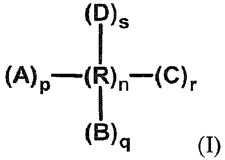 wobei
A, B, C und D reaktionsfähige Komponenten sind, die in der Lage sind, mit den freien Thiolgruppen zu reagieren, und jeweils eine oder mehrere Ladungen enthalten, wobei jede der reaktionsfähigen Komponenten A, B, C und D unabhängig eine Komponente enthält, die ausgewählt ist aus der Gruppe bestehend aus einem Vinylsulfon, einer Acrylatgruppe, einer Methacrylatgruppe, einer Styrolgruppe, einer Acrylamidgruppe, einer Methacrylamidgruppe, einer Maleatgruppe, einer Fumaratgruppe und einer Itaconatgruppe;
R(ₙ) ein Bindeglied ist, das zwei oder mehr Ladungen enthält, wobei die Ladungen den Ladungen an den reaktionsfähigen Komponenten entgegengesetzt sind, wobei R ein Monomer ist, n = 1-10 und wobei (R)ₙ kein Polymer ist und die Summe der Ladungen Null ist, und
wobei die reaktionsfähigen Komponenten ionisch an das Bindeglied gebunden sind;
wobei jedes Vorkommen von p, q, r und s unabhängig eine ganze Zahl von 0 bis 25 ist und wobei die Summe von p + q + r + s gleich oder größer als 2 ist,
in einer wirksamen Menge, um die freien Thiolgruppen kovalent zu binden.

25. Verfahren zum Setzen heller Strähnchen in Haar, Folgendes umfassend:
(a) Aufbringen einer ersten Formulierung, die ein Bleichmittel umfasst, um Haar aufzuhellen und freie Thiolgruppen zu erzeugen,
(b) Aufbringen - auf das Haar - einer zweiten Formulierung, die ein Bindemittel der Formel I umfasst 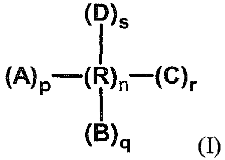 wobei
A, B, C und D reaktionsfähige Komponenten sind, die in der Lage sind, mit den freien Thiolgruppen zu reagieren, und jeweils eine oder mehrere Ladungen enthalten, wobei jede der reaktionsfähigen Komponenten A, B, C und D unabhängig eine Komponente enthält, die ausgewählt ist aus der Gruppe bestehend aus einem Vinylsulfon, einer Acrylatgruppe, einer Methacrylatgruppe, einer Styrolgruppe, einer Acrylamidgruppe, einer Methacrylamidgruppe, einer Maleatgruppe, einer Fumaratgruppe und einer Itaconatgruppe;
R(ₙ) ein Bindeglied ist, das zwei oder mehr Ladungen enthält, wobei die Ladungen den Ladungen an den reaktionsfähigen Komponenten entgegengesetzt sind, wobei R ein Monomer ist, n = 1-10 und wobei (R)ₙ kein Polymer ist und die Summe der Ladungen Null ist, und
wobei die reaktionsfähigen Komponenten ionisch an das Bindeglied gebunden sind;
wobei jedes Vorkommen von p, q, r und s unabhängig eine ganze Zahl von 0 bis 25 ist und wobei die Summe von p + q + r + s gleich oder größer als 2 ist,
in einer wirksamen Menge, um die freien Thiolgruppen kovalent zu binden.

26. Verfahren nach Anspruch 24 oder 25, wobei die Schritte (a) und (b) gleichzeitig durchgeführt werden.

27. Verfahren nach Anspruch 24 oder 25, wobei die Schritte (a) und (b) nacheinander durchgeführt werden, wobei Schritt (a) vor Schritt (b) durchgeführt wird.

28. Verfahren nach Anspruch 26, wobei vor Schritt (a) die erste Formulierung und die zweite Formulierung zusammengemischt werden.

29. Verfahren nach einem der Ansprüche 24-28, wobei die reaktionsfähigen Komponenten und die Thiolgruppen reagieren, um kovalente Kohlenstoff-Schwefel(C-S)-Bindungen auszubilden.

30. Verfahren nach einem der Ansprüche 24-29, wobei A, B, C und D gleich sind.

31. Verfahren nach einem der Ansprüche 24-29, wobei wenigstens eines von A, B, C und D anders ist als die anderen reaktionsfähigen Komponenten.

32. Verfahren nach einem der Ansprüche 24-31, wobei das Bindeglied ein polyfunktionales Molekül ist,
wobei das Bindeglied optional unabhängig substituiert ist mit einem oder mehreren Substituenten, umfassend Wasserstoff, Halogen, Cyano, Alkoxy, Alkyl, Alkenyl, Cycloalkyl, Cycloalkenyl, Aryl, Heterocycloalkyl, Heteroaryl, Amin, Hydroxy, Formyl, Acyl, Carbonsäure (-COOH), -C(O)R¹, -C(O)OR¹, Carboxylat (-COO-), primäres Amid (z. B. -CONH₂), sekundäres Amid (z. B. -CONHR¹), -C(O)NR¹R², -NR¹R², -NR¹S(O)₂R², -NR¹C(O)R², -S(O)₂R², -SR¹ und -S(O)₂NR¹R², eine Sulfinylgruppe (z. B. -SOR¹) und eine Sulfonylgruppe (z. B. -SOOR¹);
wobei R¹ und R² jeweils unabhängig Wasserstoff, Alkyl, Alkenyl, Alkinyl, Cycloalkyl, Aryl, Heterocycloalkyl und Heteroaryl sein können; wobei jedes von R¹ und R² optional unabhängig substituiert ist mit einem oder mehreren Substituenten, ausgewählt aus der Gruppe bestehend aus Halogen, Hydroxyl, Cyano, Nitro, Amino, Alkylamino, Dialkylamino, Alkyl, optional substituiert mit einem oder mehreren Halogen oder Alkoxy oder Aryloxy, Aryl, optional substituiert mit einem oder mehreren Halogen oder Alkoxy oder Alkyl oder Trihaloalkyl, Heterocycloalkyl, optional substituiert mit Aryl oder Heteroaryl oder =O oder Alkyl, optional substituiert mit Hydroxyl, Cycloalkyl, optional substituiert mit Hydroxyl, Heteroaryl, optional substituiert mit einem oder mehreren Halogen oder Alkoxy oder Alkyl oder Trihaloalkyl, Haloalkyl, Hydroxyalkyl, Carboxy, Alkoxy, Aryloxy, Alkoxycarbonyl, Aminocarbonyl, Alkylaminocarbonyl und Dialkylaminocarbonyl.

33. Verfahren nach einem der Ansprüche 24-32, wobei das Bindemittel Folgendes ist: 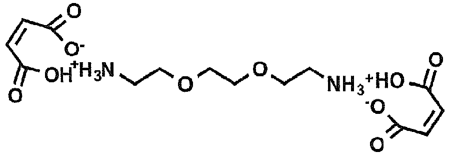 oder

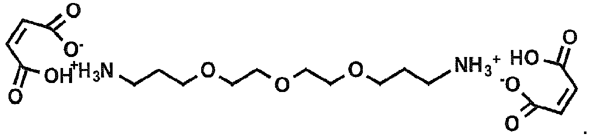

34. Verfahren nach einem der Ansprüche 24-33, wobei die zweite Formulierung ferner einen oder mehrere kosmetisch annehmbare Hilfsstoffe umfasst und
wobei der eine oder die mehreren Hilfsstoffe ausgewählt ist/sind aus der Gruppe bestehend aus Wasser, oberflächenaktiven Mitteln, Vitaminen, natürlichen Extrakten, Konservierungsmitteln, Chelatbildnern, Parfümen, Konservierungsmitteln, Antioxidationsmitteln, Proteinen, Aminosäuren, Feuchthaltemitteln, Duftstoffen, Weichmachern, Eindringmitteln, Verdickungsmitteln, Viskositätsmodifizierern, Haarfixiermitteln, Filmbildnern, Emulgatoren, Trübungsmitteln, Treibmitteln, Trägerflüssigkeiten, Trägern, Salzen, pH-Reglern, Neutralisierungsmitteln, Puffern, Haarkonditionierungsmitteln, Antistatika, Antikräuselmitteln, Antischuppenmitteln und Kombinationen derselben.

35. Verfahren nach Anspruch 34, wobei das Bindemittel in einer Menge im Bereich von 0,01 Gew.-% bis 50 Gew.-% der zweiten Formulierung, vorzugsweise von 0,01 Gew.-% bis 10 Gew.-% der zweiten Formulierung, vorhanden ist.

36. Verfahren nach einem der Ansprüche 33-35, wobei das Bindemittel in einer Menge im Bereich von 2,5 bis 3 Gew.-% der zweiten Formulierung vorhanden ist.

37. Verfahren nach einem der Ansprüche 33-36, wobei der Hilfsstoff in einer Menge im Bereich von 10 Gew.-% bis 99,99 Gew.-% der zweiten Formulierung, vorzugsweise von 50 Gew.-% bis 90 Gew.-% der zweiten Formulierung, vorhanden ist.

38. Verfahren nach einem der Ansprüche 24-37, wobei die zweite Formulierung in Form eines Gels, einer Creme, einer Lotion, eines Shampoos oder einer Pflegespülung vorliegt.

39. Verfahren nach einem der Ansprüche 24-38, wobei Schritt (b) ein oder mehrere Male wiederholt wird.

40. Verfahren nach einem der Ansprüche 24-39, ferner Folgendes umfassend:
(c) Ausspülen, Shampoonieren und/oder Pflegespülen des Haars, wobei Schritt (c) nach Schritt (b) erfolgt.

41. Verfahren nach Anspruch 40, wobei Schritt (c) innerhalb von 10 Sekunden bis 30 Minuten, vorzugsweise zwischen 1 Minute und 20 Minuten, besonders bevorzugt etwa 10 Minuten nach Schritt (b), durchgeführt wird.

42. Verfahren nach einem der Ansprüche 24-41, wobei das Färbemittel ausgewählt ist aus der Gruppe bestehend aus Mitteln zum Setzen heller Strähnchen, permanenten Färbemitteln, demi-permanenten Färbemitteln und semi-permanenten Färbemitteln.

43. Set, Folgendes umfassend:
(a) eine erste Formulierung, umfassend ein Reduktionsmittel, das in der Lage ist, Disulfidbindungen in Haar zu reduzieren, um freie Thiolgruppen zu erzeugen, und
(b) eine zweite Formulierung, umfassend ein Bindemittel in einer wirksamen Menge, um freie Thiolgruppen in Haar kovalent zu binden, wobei das Bindemittel durch die Formel I dargestellt wird: 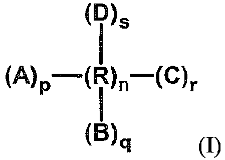 wobei
A, B, C und D reaktionsfähige Komponenten sind, die in der Lage sind, mit den freien Thiolgruppen zu reagieren, und jeweils eine oder mehrere Ladungen enthalten, wobei jede der reaktionsfähigen Komponenten A, B, C und D unabhängig eine Komponente enthält, die ausgewählt ist aus der Gruppe bestehend aus einem Vinylsulfon, einer Acrylatgruppe, einer Methacrylatgruppe, einer Styrolgruppe, einer Acrylamidgruppe, einer Methacrylamidgruppe, einer Maleatgruppe, einer Fumaratgruppe und einer Itaconatgruppe;
R(ₙ) ein Bindeglied ist, das zwei oder mehr Ladungen enthält, wobei die Ladungen den Ladungen an den reaktionsfähigen Komponenten entgegengesetzt sind, wobei R ein Monomer ist, n = 1-10 und wobei (R)ₙ kein Polymer ist und die Summe der Ladungen Null ist, und
wobei die reaktionsfähigen Komponenten ionisch an das Bindeglied gebunden sind;
wobei jedes Vorkommen von p, q, r und s unabhängig eine ganze Zahl von 0 bis 25 ist und wobei die Summe von p + q + r + s gleich oder größer als 2 ist.

44. Set nach Anspruch 43, ferner umfassend ein Shampoo, eine Pflegespülung, eine Gebrauchsanweisung, einen Entwickler, einen Mischbehälter, einen Geruchsvernichter, Handschuhe oder eine Kombination derselben.

45. Set nach einem der Ansprüche 43-44, wobei die erste Formulierung eine Bleich-, Glättungs-, Wellenerzeugungs-, Lockenerzeugungs- oder Färbeformulierung ist.

46. Set nach einem der Ansprüche 43-45, wobei A, B, C und D gleich sind.

47. Set nach einem der Ansprüche 43-46, wobei wenigstens eines von A, B, C und D anders ist als die anderen reaktionsfähigen Komponenten.

48. Set nach einem der Ansprüche 43-47, wobei das Bindeglied ein polyfunktionales Molekül ist,
wobei das Bindeglied optional unabhängig substituiert ist mit einem oder mehreren Substituenten, ausgewählt aus der Gruppe bestehend aus Wasserstoff, Halogen, Cyano, Alkoxy, Alkyl, Alkenyl, Cycloalkyl, Cycloalkenyl, Aryl, Heterocycloalkyl, Heteroaryl, Amin, Hydroxy, Formyl, Acyl, Carbonsäure (-COOH), -C(O)R¹, -C(O)OR¹, Carboxylat (-COO-), primärem Amid (z. B. -CONH₂), sekundärem Amid (z. B. -CONHR¹), -C(O)NR¹R², -NR¹R², -NR¹S(O)₂R², -NR¹C(O)R², -S(O)₂R², -SR¹ und -S(O)₂NR¹R², einer Sulfinylgruppe (z. B. -SOR¹) und einer Sulfonylgruppe (z. B. -SOOR¹);
wobei R¹ und R² jeweils unabhängig Wasserstoff, Alkyl, Alkenyl, Alkinyl, Cycloalkyl, Aryl, Heterocycloalkyl und Heteroaryl sein können; wobei jedes von R¹ und R² optional unabhängig substituiert ist mit einem oder mehreren Substituenten, ausgewählt aus der Gruppe bestehend aus Halogen, Hydroxyl, Cyano, Nitro, Amino, Alkylamino, Dialkylamino, Alkyl, optional substituiert mit einem oder mehreren Halogen oder Alkoxy oder Aryloxy, Aryl, optional substituiert mit einem oder mehreren Halogen oder Alkoxy oder Alkyl oder Trihaloalkyl, Heterocycloalkyl, optional substituiert mit Aryl oder Heteroaryl oder =O oder Alkyl, optional substituiert mit Hydroxyl, Cycloalkyl, optional substituiert mit Hydroxyl, Heteroaryl, optional substituiert mit einem oder mehreren Halogen oder Alkoxy oder Alkyl oder Trihaloalkyl, Haloalkyl, Hydroxyalkyl, Carboxy, Alkoxy, Aryloxy, Alkoxycarbonyl, Aminocarbonyl, Alkylaminocarbonyl und Dialkylaminocarbonyl.

49. Set nach einem der Ansprüche 43-48, wobei die reaktionsfähigen Komponenten und die Thiolgruppen reagieren, um kovalente Kohlenstoff-Schwefel(C-S)-Bindungen auszubilden.

50. Set nach einem der Ansprüche 43-49, wobei das Bindemittel Folgendes ist: 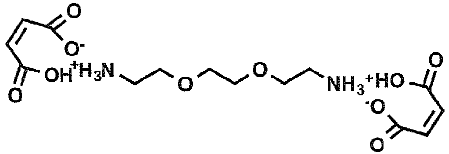 oder

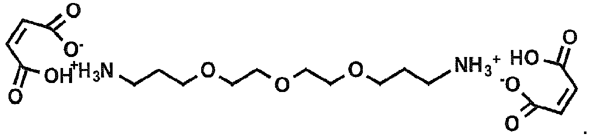

51. Set nach einem der Ansprüche 43-50, wobei die zweite Formulierung ferner einen oder mehrere kosmetisch annehmbare Hilfsstoffe umfasst, ausgewählt aus der Gruppe bestehend aus Wasser, oberflächenaktiven Mitteln, Vitaminen, natürlichen Extrakten, Konservierungsmitteln, Chelatbildnern, Parfümen, Konservierungsmitteln, Antioxidationsmitteln, Chelatbildnern, Haarfärbemitteln, Proteinen, Aminosäuren, Feuchthaltemitteln, Duftstoffen, Weichmachern, Eindringmitteln, Verdickungsmitteln, Viskositätsmodifizierern, Haarfixiermitteln, Filmbildnern, Emulgatoren, Trübungsmitteln, Treibmitteln, Trägerflüssigkeiten, Trägern, Salzen, pH-Reglern, Neutralisierungsmitteln, Puffern, Haarkonditionierungsmitteln, Antistatika, Antikräuselmitteln, Antischuppenmitteln und Kombinationen derselben.

52. Set nach einem der Ansprüche 43-51, wobei das Bindemittel in einer Menge im Bereich von 0,01 Gew.-% bis 50 Gew.-% der zweiten Formulierung vorhanden ist.

53. Set nach einem der Ansprüche 43-50, wobei der Hilfsstoff in einer Menge im Bereich von 10 Gew.-% bis 90 Gew.-% der zweiten Formulierung vorhanden ist.

54. Set nach einem der Ansprüche 43-53, wobei die erste und zweite Formulierung vorgemischt sind.

55. Set nach einem der Ansprüche 43-54, wobei die zweite Formulierung mit einem Shampoo oder einer Pflegespülung vorgemischt ist.

56. Set nach einem der Ansprüche 43-55, wobei die zweite Formulierung in Form einer Flüssigkeit, einer Lotion, einer Milch, eines Schaums, eines Sprays, eines Gels, einer Creme, eines Shampoos oder einer Pflegespülung vorliegt.

57. Set nach einem der Ansprüche 43-56, wobei die zweite Formulierung als zwei oder mehr separate Inhaltsstoffe bereitgestellt wird.

58. Set nach einem der Ansprüche 43-57, wobei das Bindemittel als ein trockenes Pulver in einer dicht verschlossenen Verpackung und der pharmazeutisch annehmbare Hilfsstoff in einem Fläschchen oder einem anderen Behälter bereitgestellt wird.

59. Set nach einem der Ansprüche 43-58, wobei:
(1) die erste Formulierung eine Bleichformulierung ist; und
(2) das Bindemittel der zweiten Formulierung Folgendes ist:

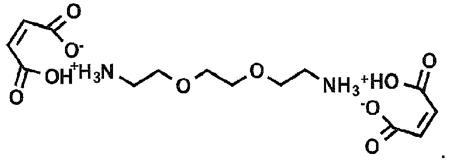

60. Set nach Anspruch 59, wobei die Bleichformulierung ferner einen Entwickler umfasst.

61. Set nach einem der Ansprüche 59-60, ferner umfassend ein Shampoo, eine Pflegespülung, eine Gebrauchsanweisung, einen Entwickler, einen Mischbehälter, einen Geruchsvernichter oder eine Kombination derselben.

62. Shampoo oder Pflegespülung, umfassend eine wirksame Menge eines Bindemittels, wobei das Bindemittel durch die Formel I dargestellt wird: 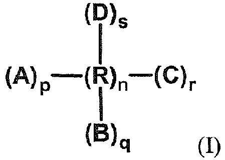 wobei
A, B, C und D reaktionsfähige Komponenten sind, die in der Lage sind, mit den freien Thiolgruppen zu reagieren, und jeweils eine oder mehrere Ladungen enthalten, wobei jede der reaktionsfähigen Komponenten A, B, C und D unabhängig eine Komponente enthält, die ausgewählt ist aus der Gruppe bestehend aus einem Vinylsulfon, einer Acrylatgruppe, einer Methacrylatgruppe, einer Styrolgruppe, einer Acrylamidgruppe, einer Methacrylamidgruppe, einer Maleatgruppe, einer Fumaratgruppe und einer Itaconatgruppe;
R(ₙ) ein Bindeglied ist, das zwei oder mehr Ladungen enthält, wobei die Ladungen den Ladungen an den reaktionsfähigen Komponenten entgegengesetzt sind, wobei R ein Monomer ist, n = 1-10 und wobei (R)ₙ kein Polymer ist und die Summe der Ladungen Null ist, und
wobei die reaktionsfähigen Komponenten ionisch an das Bindeglied gebunden sind;
wobei jedes Vorkommen von p, q, r und s unabhängig eine ganze Zahl von 0 bis 25 ist und wobei die Summe von p + q + r + s gleich oder größer als 2 ist;
mit der Maßgabe, dass das Bindemittel der Formel I kein Trimaleatsalz von H₂N-(CH₂)₃-N¹(G)-(CH₂)₄-NH₂ ist,
wobei G ein Substituent ist, der an die sekundäre Amingruppe von Spermidin gebunden ist, ausgewählt aus gesättigten oder ungesättigten, linearen oder verzweigten Alkylgruppen, die aus 1 bis 6 Kohlenstoffatomen ausgebildet sind, wobei ein oder mehrere Kohlenstoffatome optional durch Fluor ersetzt ist/sind, konkret Methyl, Ethyl, Trifluormethyl, Trifluorethyl, Propyl, Isopropyl, Butyl, Isobutyl, Pentyl, Hexyl, Ethylen, Vinyl, Propylen, Butylen; Aryl- oder Aryl-Alkyl-Gruppen, wie etwa Phenyl, Naphthyl, Benzyl, Tolyl, wobei ein oder mehrere Kohlenstoffatome optional durch Fluor ersetzt ist/sind und wobei die Aryl-Alkyl-Gruppen gesättigte oder ungesättigte, lineare oder verzweigte Alkylgruppen, die durch 1 bis 6 Kohlenstoffatome ausgebildet sind, umfassen, wobei ein oder mehrere Kohlenstoffatome optional durch Fluor ersetzt ist/sind, konkret Methyl, Ethyl, Trifluormethyl, Trifluorethyl, Propyl, Isopropyl, Butyl, Isobutyl, Pentyl, Hexyl, Ethylen, Vinyl, Propylen, Butylen; gesättigte oder ungesättigte Cycloalkylgruppen, die durch 3 bis 8 Kohlenstoffatome ausgebildet sind, die optional ersetzt sind durch gesättigte oder ungesättigte, lineare oder verzweigte Alkylgruppen, die durch 1 bis 6 Kohlenstoffatome ausgebildet sind, wobei ein oder mehrere Kohlenstoffatome optional durch Fluor ersetzt ist/sind, konkret Methyl, Ethyl, Trifluormethyl, Trifluorethyl, Propyl, Isopropyl, Butyl, Isobutyl, Pentyl, Hexyl, Ethylen, Vinyl, Propylen, Butylen.

63. Shampoo oder Pflegespülung nach Anspruch 62, wobei die reaktionsfähigen Komponenten und die Thiolgruppen reagieren, um kovalente Kohlenstoff-Schwefel(C-S)-Bindungen auszubilden.

64. Shampoo oder Pflegespülung nach einem der Ansprüche 62-63, wobei A, B, C und D gleich sind.

65. Shampoo oder Pflegespülung nach einem der Ansprüche 62-63, wobei wenigstens eines von A, B, C und D anders ist als die anderen reaktionsfähigen Komponenten.

66. Shampoo oder Pflegespülung nach einem der Ansprüche 62-65, wobei das Bindeglied ein polyfunktionales Molekül ist,
wobei das Bindeglied optional unabhängig substituiert ist mit einem oder mehreren Substituenten, ausgewählt aus der Gruppe bestehend aus Wasserstoff, Halogen, Cyano, Alkoxy, Alkyl, Alkenyl, Cycloalkyl, Cycloalkenyl, Aryl, Heterocycloalkyl, Heteroaryl, Amin, Hydroxy, Formyl, Acyl, Carbonsäure (-COOH), -C(O)R¹, -C(O)OR¹, Carboxylat (-COO-), primärem Amid (z. B. -CONH₂), sekundärem Amid (z. B. -CONHR¹), -C(O)NR¹R², -NR¹R², -NR¹S(O)₂R², -NR¹C(O)R², -S(O)₂R², -SR¹ und -S(O)₂NR¹R², einer Sulfinylgruppe (z. B. -SOR¹¹) und einer Sulfonylgruppe (z. B. -SOOR¹);
wobei R¹ und R² jeweils unabhängig Wasserstoff, Alkyl, Alkenyl, Alkinyl, Cycloalkyl, Aryl, Heterocycloalkyl und Heteroaryl sein können;
wobei jedes von R¹ und R² optional unabhängig substituiert ist mit einem oder mehreren Substituenten, ausgewählt aus der Gruppe bestehend aus Halogen, Hydroxyl, Cyano, Nitro, Amino, Alkylamino, Dialkylamino, Alkyl, optional substituiert mit einem oder mehreren Halogen oder Alkoxy oder Aryloxy, Aryl, optional substituiert mit einem oder mehreren Halogen oder Alkoxy oder Alkyl oder Trihaloalkyl, Heterocycloalkyl, optional substituiert mit Aryl oder Heteroaryl oder =O oder Alkyl, optional substituiert mit Hydroxyl, Cycloalkyl, optional substituiert mit Hydroxyl, Heteroaryl, optional substituiert mit einem oder mehreren Halogen oder Alkoxy oder Alkyl oder Trihaloalkyl, Haloalkyl, Hydroxyalkyl, Carboxy, Alkoxy, Aryloxy, Alkoxycarbonyl, Aminocarbonyl, Alkylaminocarbonyl und Dialkylaminocarbonyl.

67. Shampoo oder Pflegespülung nach einem der Ansprüche 62-66, wobei das Bindemittel Folgendes ist: 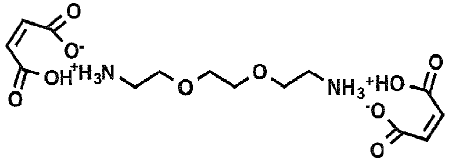 oder

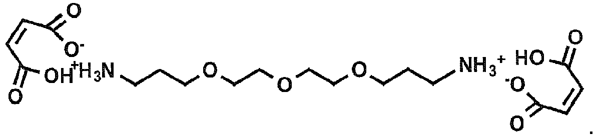

68. Shampoo oder Pflegespülung nach einem der Ansprüche 62-67, ferner umfassend einen oder mehrere kosmetisch annehmbare Hilfsstoffe, wobei der eine oder die mehreren Hilfsstoffe ausgewählt ist/sind aus der Gruppe bestehend aus Wasser, oberflächenaktiven Mitteln, Vitaminen, natürlichen Extrakten, Konservierungsmitteln, Chelatbildnern, Parfümen, Konservierungsmitteln, Antioxidationsmitteln, Chelatbildnern, Haarfärbemitteln, Proteinen, Aminosäuren, Feuchthaltemitteln, Duftstoffen, Weichmachern, Eindringmitteln, Verdickungsmitteln, Viskositätsmodifizierern, Haarfixiermitteln, Filmbildnern, Emulgatoren, Trübungsmitteln, Treibmitteln, Trägerflüssigkeiten, Trägern, Salzen, pH-Reglern, Neutralisierungsmitteln, Puffern, Haarkonditionierungsmitteln, Antistatika, Antikräuselmitteln, Antischuppenmitteln und Kombinationen derselben.

69. Shampoo oder Pflegespülung nach Anspruch 68, wobei das Bindemittel in einer Menge im Bereich von 0,01 Gew.-% bis 50 Gew.-% der Formulierung vorhanden ist.

70. Shampoo oder Pflegespülung nach einem der Ansprüche 68-69, wobei das Bindemittel in einer Menge im Bereich von 2,5 bis 3 Gew.-% der Formulierung vorhanden ist.

## Revendications

1. Procédé de traitement des cheveux, dans lequel les cheveux comprennent deux ou plusieurs groupes thiols libres, comprenant :
(a) l'application sur les cheveux d'une formulation comprenant un agent liant de Formule I 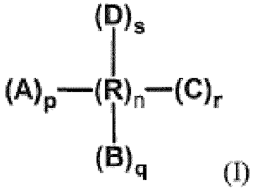 dans laquelle
A, B, C et D sont des groupements réactifs capables de réagir avec les groupes thiols libres contenant chacun une ou plusieurs charges, où chacun des groupements réactifs A, B, C et D contient indépendamment un groupement choisi dans le groupe constitué par une vinylsulfone, un groupe acrylate, un groupe méthacrylate, un groupe styrène, un groupe acrylamide, un groupe méthacrylamide, un groupe maléate, un groupe fumarate et un groupe itaconate ;
(R)ₙ est un lieur contenant deux ou plusieurs charges, où les charges sont opposées aux charges sur les groupements réactifs, où R est un monomère, n = 1 à 10, et où (R)ₙ n'est pas un polymère, et
la somme des charges est nulle, et
où les groupements réactifs sont liés de manière ionique au lieur ;
où chaque occurrence de p, q, r et s est indépendamment un nombre entier compris entre 0 et 25, et où la somme de p + q + r + s est égale ou supérieure à 2, dans une quantité efficace pour lier de manière covalente les groupes thiols libres ;
à condition que l'agent liant de Formule I ne soit pas un sel de trimaléate de H₂N-(CH₂)₃-N¹(G)-(CH₂)₄-NH₂
où G est un substituant lié au groupe amine secondaire de spermidine, choisi parmi les groupes alkyles saturés ou insaturés, linéaires ou ramifiés constitués de 1 à 6 atomes de carbone, où un ou plusieurs atomes de carbone sont facultativement remplacés par du fluor, à savoir du méthyle, de l'éthyle, du trifluorométhyle, du trifluoroéthyle, du propyle, de l'isopropyle, du butyle, de l'isobutyle, du pentyle, de l'hexyle, de l'éthylène, du vinyle, du propylène, du butylène ; les groupes aryles ou arylalkyles, tels que le phényle, le naphtyle, le benzyle, le tolyle, où un ou plusieurs atomes de carbone sont facultativement remplacés par du fluor, et où lesdits groupes arylalkyles comprennent les groupes alkyles saturés ou insaturés, linéaires ou ramifiés constitués de 1 à 6 atomes de carbone, où un ou plusieurs atomes de carbone sont facultativement remplacés par du fluor, à savoir du méthyle, de l'éthyle, du trifluorométhyle, du trifluoroéthyle, du propyle, de l'isopropyle, du butyle, de l'isobutyle, du pentyle, de l'hexyle, de l'éthylène, du vinyle, du propylène, du butylène ; les groupes cycloalkyles saturés ou insaturés constitués par 3 à 8 atomes de carbone qui sont facultativement remplacés par des groupes alkyles saturés ou insaturés, linéaires ou ramifiés constitués de 1 à 6 atomes de carbone, où un ou plusieurs atomes de carbone sont facultativement remplacés par du fluor, à savoir du méthyle, de l'éthyle, du trifluorométhyle, du trifluoroéthyle, du propyle, de l'isopropyle, du butyle, de l'isobutyle, du pentyle, de l'hexyle, de l'éthylène, du vinyle, du propylène, du butylène.

2. Procédé selon la revendication 1, dans lequel les groupements réactifs et les groupes thiols réagissent pour former des liaisons covalentes carbone-soufre (C-S).

3. Procédé selon l'une quelconque des revendications 1 à 2, dans lequel A, B, C et D sont identiques.

4. Procédé selon l'une quelconque des revendications 1 à 2, dans lequel au moins l'un de A, B, C et D est différent des autres groupements réactifs.

5. Procédé selon l'une quelconque des revendications 1 à 4, dans lequel le lieur est une molécule polyfonctionnelle, et dans lequel le lieur est facultativement indépendamment substitué par un ou plusieurs substituants comprenant l'hydrogène, l'halogène, le cyano, l'alcoxy, l'alkyle, l'alcényle, le cycloalkyle, le cycloalcényle, l'aryle, l'hétérocycloalkyle, l'hétéroaryle, l'amine, l'hydroxy, le formyle, l'acyle, l'acide carboxylique (-COOH), -C(O)R¹, - C(O)OR¹, le carboxylate (-COO⁻), l'amide primaire (par exemple, -CONH₂), l'amide secondaire (par exemple, -CONHR¹), -C(O)NR¹R², -NR¹R², -NR¹S(O)₂R², -NR¹C(O)R², - S(O)₂R², -SR¹, -S(O)₂NR¹R², le groupe sulfinyle (par ex., - SOR¹) et le groupe sulfonyle (par ex., -SOOR¹) ;
où R¹ et R² peuvent être chacun indépendamment de l'hydrogène, de l'alkyle, de l'alcényle, de l'alcynyle, du cycloalkyle, de l'aryle, de l'hétérocycloalkyle et de l'hétéroaryle ; où chacun de R¹ et R² est facultativement indépendamment substitué par un ou plusieurs substituants choisis dans le groupe constitué par l'halogène, l'hydroxyle, le cyano, le nitro, l'amino, l'alkylamino, le dialkylamino, l'alkyle facultativement substitué par un ou plusieurs de l'halogène, de l'alcoxy ou de l'aryloxy, l'aryle facultativement substitué par un ou plusieurs de l'halogène, de l'alcoxy, de l'alkyle ou du trihaloalkyle, l'hétérocycloalkyle facultativement substitué par l'aryle, l'hétéroaryle ou =O, ou l'alkyle facultativement substitué par l'hydroxyle, le cycloalkyle facultativement substitué par l'hydroxyle, l'hétéroaryle facultativement substitué par un ou plusieurs de l'halogène, de l'alcoxy, de l'alkyle ou du trihaloalkyle, de l'haloalkyle, de l'hydroxyalkyle, du carboxy, de l'alcoxy, de l'aryloxy, de l'alcoxycarbonyle, de l'aminocarbonyle, de l'alkylaminocarbonyle et du dialkylaminocarbonyle.

6. Procédé selon l'une quelconque des revendications 1 à 5, dans lequel l'agent liant est : 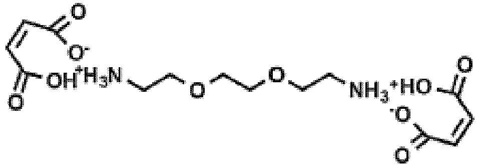 ou

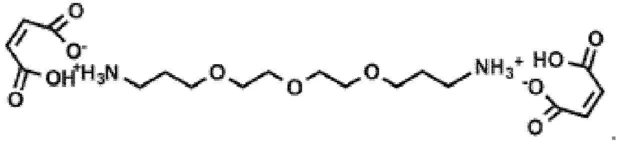

7. Procédé selon l'une quelconque des revendications 1 à 6, dans lequel les groupements réactifs A, B, C et D contiennent indépendamment un groupement choisi dans le groupe constitué par la vinylsulfone, un groupe styrène, un groupe acrylamide, un groupe méthacrylamide, un groupe maléate et un groupe fumarate.

8. Procédé selon l'une quelconque des revendications 1 à 7, dans lequel le poids moléculaire de l'agent liant est inférieur à environ 500 Daltons.

9. Procédé selon l'une quelconque des revendications 1 à 8, dans lequel la formulation comprend en outre un ou plusieurs excipients cosmétiquement acceptables,
dans lequel le ou les excipients sont choisis dans le groupe constitué par l'eau, les tensioactifs, les vitamines, les extraits naturels, les conservateurs, les agents chélatants, les parfums, les conservateurs, les antioxydants, les agents chélatants, les colorants capillaires, les protéines, les acides aminés, les humectants, les fragrances, les émollients, les pénétrants, les épaississants, les modificateurs de viscosité, les mousses coiffantes, les agents filmogènes, les émulsifiants, les opacifiants, les propulseurs, les véhicules liquides, les vecteurs, les sels, les agents d'ajustement du pH, les agents neutralisants, les tampons, les démêlants, les agents antistatiques, les agents anti-frisottis, les agents antipelliculaires et les combinaisons de ceux-ci.

10. Procédé selon la revendication 9, dans lequel l'agent liant est présent dans une quantité comprise entre 0,01 % en poids et 50 % en poids de la formulation.

11. Procédé selon l'une quelconque des revendications 9 et 10, dans lequel l'agent liant constitue environ 3 % en poids de la formulation.

12. Procédé selon l'une quelconque des revendications 9 à 11, dans lequel l'excipient est présent dans une quantité comprise entre 10 % en poids et 90 % en poids de la formulation.

13. Procédé selon l'une quelconque des revendications 1 à 12, dans lequel la formulation est sous forme de gel, de crème, de lotion, de shampoing ou d'après-shampoing.

14. Procédé selon l'une quelconque des revendications 1 à 13, dans lequel l'étape (a) est répétée une ou plusieurs fois.

15. Procédé selon l'une quelconque des revendications 1 à 14, dans lequel l'étape (a) est répétée 1 minute à 20 minutes après la première application de la formulation.

16. Procédé selon l'une quelconque des revendications 1 à 15, comprenant en outre :
(b) le rinçage, le shampouinage et/ou le conditionnement des cheveux, où l'étape (b) a lieu après l'étape (a).

17. Procédé selon la revendication 16, dans lequel l'étape (b) est effectuée 10 secondes à 30 secondes après l'étape (a).

18. Procédé selon l'une quelconque des revendications 1 à 17, comprenant en outre l'étape consistant à :
appliquer une première formulation comprenant un agent réducteur capable de réduire les liaisons disulfures dans les cheveux pour produire des groupes thiols libres,
dans lequel l'étape est effectuée avant d'appliquer la formulation comprenant l'agent liant.

19. Procédé selon la revendication 18, dans lequel l'agent réducteur est choisi dans le groupe constitué par l'acide thioglycolique et ses sels et esters dérivés, l'acide thiolactique et ses sels et esters dérivés, la cystéine et ses dérivés, la cystéamine et ses dérivés, les sulfites inorganiques, le métabisulfite de sodium, d'autres bisulfites inorganiques, le dithiothréitol, le dithioérythritol, les phosphines organiques et les défrisants japonais.

20. Procédé selon l'une quelconque des revendications 1 à 19, dans lequel les thiols restent liés pendant au moins une semaine.

21. Procédé selon l'une quelconque des revendications 1 à 20, dans lequel le poids moléculaire de l'agent liant est inférieur à 500 Daltons.

22. Formulation constituée d'un agent liant, d'un solvant aqueux et d'un ou plusieurs conservateurs, stabilisants ou combinaisons de ceux-ci,
dans lequel l'agent liant est représenté par la Formule I : 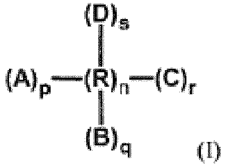 dans laquelle
A, B, C et D sont des groupements réactifs capables de réagir avec les groupes thiols libres contenant chacun une ou plusieurs charges, où chacun des groupements réactifs A, B, C et D contient indépendamment un groupement choisi dans le groupe constitué par une vinylsulfone, un groupe acrylate, un groupe méthacrylate, un groupe styrène, un groupe acrylamide, un groupe méthacrylamide, un groupe maléate, un groupe fumarate et un groupe itaconate ;
(R)ₙ est un lieur contenant deux ou plusieurs charges, où les charges sont opposées aux charges sur les groupements réactifs, où R est un monomère, n = 1 à 10, et où (R)ₙ n'est pas un polymère, et la somme des charges est nulle, et
où les groupements réactifs sont liés de manière ionique au lieur ;
où chaque occurrence de p, q, r et s est indépendamment un nombre entier compris entre 0 et 25, et où la somme de p + q + r + s est égale ou supérieure à 2.

23. Formulation selon la revendication 22, dans laquelle l'agent liant est : 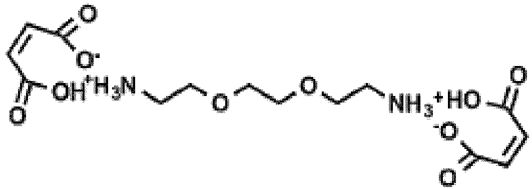 ou

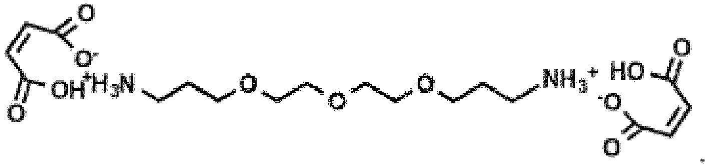

24. Procédé de coloration des cheveux comprenant :
(a) l'application d'une première formulation comprenant un colorant capillaire et un agent réducteur capable de réduire les liaisons disulfures dans les cheveux pour produire des groupes thiols libres,
(b) l'application sur les cheveux d'une deuxième formulation comprenant un agent liant de Formule I 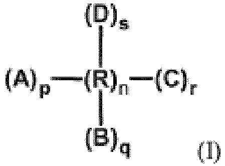 dans laquelle
A, B, C et D sont des groupements réactifs capables de réagir avec les groupes thiols libres contenant chacun une ou plusieurs charges, où chacun des groupements réactifs A, B, C et D contient indépendamment un groupement choisi dans le groupe constitué par une vinylsulfone, un groupe acrylate, un groupe méthacrylate, un groupe styrène, un groupe acrylamide, un groupe méthacrylamide, un groupe maléate, un groupe fumarate et un groupe itaconate ;
(R)ₙ est un lieur contenant deux ou plusieurs charges, où les charges sont opposées aux charges sur les groupements réactifs, où R est un monomère, n = 1 à 10, et où (R)ₙ n'est pas un polymère, et la somme des charges est nulle, et
où les groupements réactifs sont liés de manière ionique au lieur ;
où chaque occurrence de p, q, r et s est indépendamment un nombre entier compris entre 0 et 25, et où la somme de p + q + r + s est égale ou supérieure à 2,
dans une quantité efficace pour lier de manière covalente les groupes thiols libres.

25. Procédé de balayage des cheveux comprenant :
(a) l'application d'une première formulation comprenant l'éclaircissant pour éclaircir les cheveux et produire des groupes thiols libres,
(b) l'application sur les cheveux d'une deuxième formulation comprenant un agent liant de Formule I 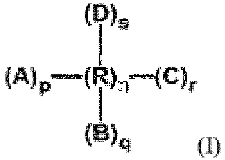 dans laquelle
A, B, C et D sont des groupements réactifs capables de réagir avec les groupes thiols libres contenant chacun une ou plusieurs charges, où chacun des groupements réactifs A, B, C et D contient indépendamment un groupement choisi dans le groupe constitué par une vinylsulfone, un groupe acrylate, un groupe méthacrylate, un groupe styrène, un groupe acrylamide, un groupe méthacrylamide, un groupe maléate, un groupe fumarate et un groupe itaconate ;
(R)ₙ est un lieur contenant deux ou plusieurs charges, où les charges sont opposées aux charges sur les groupements réactifs, où R est un monomère, n = 1 à 10, et où (R)ₙ n'est pas un polymère, et la somme des charges est nulle, et
où les groupements réactifs sont liés de manière ionique au lieur ;
où chaque occurrence de p, q, r et s est indépendamment un nombre entier compris entre 0 et 25, et où la somme de p + q + r + s est égale ou supérieure à 2,
dans une quantité efficace pour lier de manière covalente les groupes thiols libres.

26. Procédé selon la revendication 24 ou 25, dans lequel les étapes (a) et (b) sont effectuées simultanément.

27. Procédé selon la revendication 24 ou 25, dans lequel les étapes (a) et (b) sont effectuées séquentiellement, l'étape (a) étant réalisée avant l'étape (b).

28. Procédé selon la revendication 26, dans lequel, avant l'étape (a), la première formulation et la deuxième formulation sont mélangées.

29. Procédé selon l'une quelconque des revendications 24 à 28, dans lequel les groupements réactifs et les groupes thiols réagissent pour former des liaisons covalentes carbone-soufre (C-S).

30. Procédé selon l'une quelconque des revendications 24 à 29, dans lequel A, B, C et D sont identiques.

31. Procédé selon l'une quelconque des revendications 24 à 29, dans lequel au moins l'un de A, B, C et D est différent des autres groupements réactifs.

32. Procédé selon l'une quelconque des revendications 24 à 31, dans lequel le lieur est une molécule polyfonctionnelle,
dans lequel le lieur est facultativement indépendamment substitué par un ou plusieurs substituants comprenant l'hydrogène, l'halogène, le cyano, l'alcoxy, l'alkyle, l'alcényle, le cycloalkyle, le cycloalcényle, l'aryle, l'hétérocycloalkyle, l'hétéroaryle, l'amine, l'hydroxy, le formyle, l'acyle, l'acide carboxylique (-COOH), -C(O)R¹, -C(O)OR¹, le carboxylate (-COO⁻), l'amide primaire (par ex., -CONH₂), l'amide secondaire (par ex., - CONHR¹), -C(O)NR¹R², -NR¹R², -NR¹S(O)₂R², -NR¹C(O)R², -S(O)₂R², -SR¹, -S(O)₂NR¹R², le groupe sulfinyle (par ex., - SOR¹) et le groupe sulfonyle (par ex., -SOOR¹) ;
où R¹ et R² peuvent être chacun indépendamment de l'hydrogène, de l'alkyle, de l'alcényle, de l'alcynyle, du cycloalkyle, de l'aryle, de l'hétérocycloalkyle et de l'hétéroaryle ; où chacun de R¹ et R² est facultativement indépendamment substitué par un ou plusieurs substituants choisis dans le groupe constitué par l'halogène, l'hydroxyle, le cyano, le nitro, l'amino, l'alkylamino, le dialkylamino, l'alkyle facultativement substitué par un ou plusieurs de l'halogène, de l'alcoxy ou de l'aryloxy, l'aryle facultativement substitué par un ou plusieurs de l'halogène, de l'alcoxy, de l'alkyle ou du trihaloalkyle, l'hétérocycloalkyle facultativement substitué par l'aryle, l'hétéroaryle ou =O, ou l'alkyle facultativement substitué par l'hydroxyle, le cycloalkyle facultativement substitué par l'hydroxyle, l'hétéroaryle facultativement substitué par un ou plusieurs de l'halogène, de l'alcoxy, de l'alkyle, du trihaloalkyle, de l'haloalkyle, de l'hydroxyalkyle, du carboxy, de l'alcoxy, de l'aryloxy, de l'alcoxycarbonyle, de l'aminocarbonyle, de l'alkylaminocarbonyle et du dialkylaminocarbonyle.

33. Procédé selon l'une quelconque des revendications 24 à 32, dans lequel l'agent liant est : 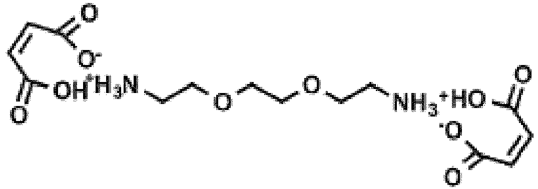 ou

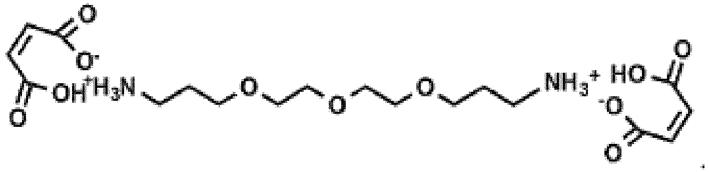

34. Procédé selon l'une quelconque des revendications 24 à 33, dans lequel la deuxième formulation comprend en outre un ou plusieurs excipients cosmétiquement acceptables, et
dans lequel le ou les excipients sont choisis dans le groupe constitué par l'eau, les tensioactifs, les vitamines, les extraits naturels, les conservateurs, les agents chélatants, les parfums, les conservateurs, les antioxydants, les protéines, les acides aminés, les humectants, les fragrances, les émollients, les pénétrants, les épaississants, les modificateurs de viscosité, les mousses coiffantes, les agents filmogènes, les émulsifiants, les opacifiants, les propulseurs, les véhicules liquides, les vecteurs, les sels, les agents d'ajustement du pH, les agents neutralisants, les tampons, les démêlants, les agents antistatiques, les agents anti-frisottis, les agents antipelliculaires et les combinaisons de ceux-ci.

35. Procédé selon la revendication 34, dans lequel l'agent liant est présent dans une quantité comprise entre 0,01 % en poids et 50 % en poids de la deuxième formulation, de préférence entre 0,01 % en poids et 10 % en poids de la deuxième formulation.

36. Procédé selon l'une quelconque des revendications 33 à 35, dans lequel l'agent liant est présent dans une quantité comprise entre environ 2,5 et 3 % en poids de la deuxième formulation.

37. Procédé selon l'une quelconque des revendications 33 à 36, dans lequel l'excipient est présent dans une quantité comprise entre 10 % en poids et 99,99 % en poids de la deuxième formulation, de préférence entre 50 % en poids et 90 % en poids de la deuxième formulation.

38. Procédé selon l'une quelconque des revendications 24 à 37, dans lequel la deuxième formulation est sous forme de gel, de crème, de lotion, de shampoing ou d'après-shampoing.

39. Procédé selon l'une quelconque des revendications 24 à 38, dans lequel l'étape (b) est répétée une ou plusieurs fois.

40. Procédé selon l'une quelconque des revendications 24 à 39, comprenant en outre :
(c) le rinçage, le shampouinage et/ou le conditionnement des cheveux, où l'étape (c) a lieu après l'étape (b).

41. Procédé selon la revendication 40, dans lequel l'étape (c) est effectuée 10 secondes à 30 secondes, de préférence entre 1 minute et 20 minutes, plus préférablement environ 10 minutes après l'étape (b).

42. Procédé selon l'une quelconque des revendications 24 à 41, dans lequel le colorant est choisi dans le groupe constitué par les éclaircissants, les colorants permanents, les colorants demi-permanents et les colorants semi-permanents.

43. Kit comprenant :
(a) une première formulation comprenant un agent réducteur capable de réduire les liaisons disulfures dans les cheveux pour produire des groupes thiols libres, et
(b) une deuxième formulation comprenant un agent liant dans une quantité efficace pour lier de façon covalente les groupes thiols libres dans les cheveux,
dans lequel l'agent liant est représenté par la Formule I : 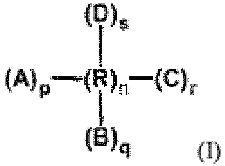 dans laquelle
A, B, C et D sont des groupements réactifs capables de réagir avec les groupes thiols libres contenant chacun une ou plusieurs charges, où chacun des groupements réactifs A, B, C et D contient indépendamment un groupement choisi dans le groupe constitué par une vinylsulfone, un groupe acrylate, un groupe méthacrylate, un groupe styrène, un groupe acrylamide, un groupe méthacrylamide, un groupe maléate, un groupe fumarate et un groupe itaconate ;
(R)ₙ est un lieur contenant deux ou plusieurs charges, où les charges sont opposées aux charges sur les groupements réactifs, où R est un monomère, n = 1 à 10, et où (R)ₙ n'est pas un polymère, et la somme des charges est nulle, et
où les groupements réactifs sont liés de manière ionique au lieur ;
où chaque occurrence de p, q, r et s est indépendamment un nombre entier compris entre 0 et 25, et où la somme de p + q + r + s est égale ou supérieure à 2.

44. Kit selon la revendication 43, comprenant en outre un shampoing, un après-shampoing, une notice d'utilisation, un révélateur, un récipient de mélange, un anti-odeur, des gants ou une combinaison de ceux-ci.

45. Kit selon l'une quelconque des revendications 43 et 44, dans lequel la première formulation est une formulation éclaircissante, lissante, ondulante, frisante ou colorante.

46. Kit selon l'une quelconque des revendications 43 à 45, dans lequel A, B, C et D sont identiques.

47. Kit selon l'une quelconque des revendications 43 à 46, dans lequel au moins l'un de A, B, C et D est différent des autres groupements réactifs.

48. Kit selon l'une quelconque des revendications 43 à 47, dans lequel le lieur est une molécule polyfonctionnelle,
dans lequel le lieur est facultativement indépendamment substitué par un ou plusieurs substituants comprenant l'hydrogène, l'halogène, le cyano, l'alcoxy, l'alkyle, l'alcényle, le cycloalkyle, le cycloalcényle, l'aryle, l'hétérocycloalkyle, l'hétéroaryle, l'amine, l'hydroxy, le formyle, l'acyle, l'acide carboxylique (-COOH), -C(O)R¹, -C(O)OR¹, le carboxylate (-COO⁻), l'amide primaire (par ex., -CONH₂), l'amide secondaire (par ex.,-CONHR¹), -C(O)NR¹R², -NR¹R², -NR¹S(O)₂R², -NR¹C(O)R², -S(O)₂R², -SR¹, -S(O)₂NR¹R², le groupe sulfinyle (par ex., - SOR¹) et le groupe sulfonyle (par ex., -SOOR¹) ;
où R¹ et R² peuvent être chacun indépendamment de l'hydrogène, de l'alkyle, de l'alcényle, de l'alcynyle, du cycloalkyle, de l'aryle, de l'hétérocycloalkyle et de l'hétéroaryle ; où chacun de R¹ et R² est facultativement indépendamment substitué par un ou plusieurs substituants choisis dans le groupe constitué par l'halogène, l'hydroxyle, le cyano, le nitro, l'amino, l'alkylamino, le dialkylamino, l'alkyle facultativement substitué par un ou plusieurs de l'halogène, de l'alcoxy ou de l'aryloxy, l'aryle facultativement substitué par un ou plusieurs de l'halogène, de l'alcoxy, de l'alkyle ou du trihaloalkyle, l'hétérocycloalkyle facultativement substitué par l'aryle, l'hétéroaryle ou =O, ou l'alkyle facultativement substitué par l'hydroxyle, le cycloalkyle facultativement substitué par l'hydroxyle, l'hétéroaryle facultativement substitué par un ou plusieurs de l'halogène, de l'alcoxy, de l'alkyle ou du trihaloalkyle, de l'haloalkyle, de l'hydroxyalkyle, du carboxy, de l'alcoxy, de l'aryloxy, de l'alcoxycarbonyle, de l'aminocarbonyle, de l'alkylaminocarbonyle et du dialkylaminocarbonyle.

49. Kit selon l'une quelconque des revendications 43 à 48, dans lequel les groupements réactifs et les groupes thiols réagissent pour former des liaisons covalentes carbone-soufre (C-S).

50. Kit selon l'une quelconque des revendications 43 à 49, dans lequel l'agent liant est : 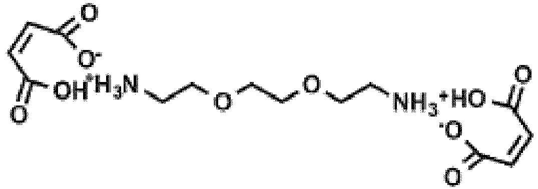 ou

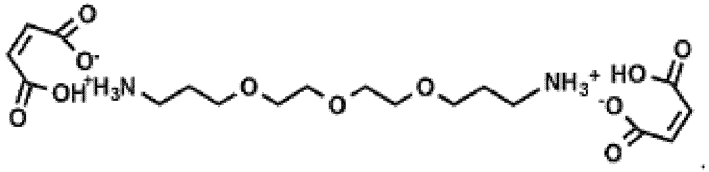

51. Kit selon l'une quelconque des revendications 43 à 50, dans lequel la deuxième formulation comprend en outre un ou plusieurs excipients cosmétiquement acceptables choisis dans le groupe constitué par l'eau, les tensioactifs, les vitamines, les extraits naturels, les conservateurs, les agents chélatants, les parfums, les conservateurs, les antioxydants, les agents chélatants, les colorants capillaires, les protéines, les acides aminés, les humectants, les fragrances, les émollients, les pénétrants, les épaississants, les modificateurs de viscosité, les mousses coiffantes, les agents filmogènes, les émulsifiants, les opacifiants, les propulseurs, les véhicules liquides, les vecteurs, les sels, les agents d'ajustement du pH, les agents neutralisants, les tampons, les conditionneurs, les agents antistatiques, les agents anti-frisottis, les agents antipelliculaires et les combinaisons de ceux-ci.

52. Kit selon l'une quelconque des revendications 43 à 51, dans lequel l'agent liant est présent dans une quantité comprise entre 0,01 % en poids et 50 % en poids de la deuxième formulation.

53. Kit selon l'une quelconque des revendications 43 à 50, dans lequel l'excipient est présent dans une quantité comprise entre 10 % en poids et 90 % en poids de la deuxième formulation.

54. Kit selon l'une quelconque des revendications 43 à 53, dans lequel les première et deuxième formulations sont pré-mélangées.

55. Kit selon l'une quelconque des revendications 43 à 54, dans lequel la deuxième formulation est pré-mélangée avec un shampoing ou un après-shampoing.

56. Kit selon l'une quelconque des revendications 43 à 55, dans lequel la deuxième formulation est sous forme de liquide, de lotion, de lait, de mousse, de spray, de gel, de crème, de shampoing ou de après-shampoing.

57. Kit selon l'une quelconque des revendications 43 à 56, dans lequel la deuxième formulation est fournie sous forme de deux ou plusieurs ingrédients séparés.

58. Kit selon l'une quelconque des revendications 43 à 57, dans lequel l'agent liant est fourni sous forme de poudre sèche dans un emballage étanche et l'excipient pharmaceutiquement acceptable est fourni dans un flacon ou un autre récipient.

59. Kit selon l'une quelconque des revendications 43 à 58, dans lequel :
(1) la première formulation est une formulation éclaircissante ; et
(2) l'agent liant de la deuxième formulation est

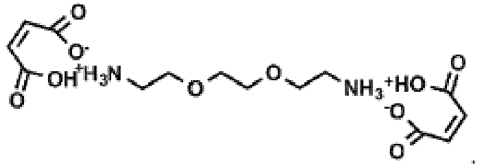

60. Kit selon la revendication 59, dans lequel la formulation éclaircissante comprend en outre un révélateur.

61. Kit selon l'une quelconque des revendications 59 et 60, comprenant en outre un shampoing, un après-shampoing, une notice d'utilisation, un révélateur, un récipient de mélange, un anti-odeur ou une combinaison de ceux-ci.

62. Shampoing ou démêlant comprenant une quantité efficace d'un agent liant, dans lequel l'agent liant est représenté par la Formule I : 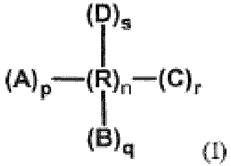 dans laquelle
A, B, C et D sont des groupements réactifs capables de réagir avec les groupes thiols libres contenant chacun une ou plusieurs charges, où chacun des groupements réactifs A, B, C et D contient indépendamment un groupement choisi dans le groupe constitué par une vinylsulfone, un groupe acrylate, un groupe méthacrylate, un groupe styrène, un groupe acrylamide, un groupe méthacrylamide, un groupe maléate, un groupe fumarate et un groupe itaconate ;
(R)ₙ est un lieur contenant deux ou plusieurs charges, où les charges sont opposées aux charges sur les groupements réactifs, où R est un monomère, n = 1 à 10, et où (R)ₙ n'est pas un polymère, et la somme des charges est nulle, et
où les groupements réactifs sont liés de manière ionique au lieur ;
où chaque occurrence de p, q, r et s est indépendamment un nombre entier compris entre 0 et 25, et où la somme de p + q + r + s est égale ou supérieure à 2 ;
à condition que l'agent liant de Formule I ne soit pas un sel de trimaléate de H₂N-(CH₂)₃-N¹(G)-(CH₂)₄-NH₂
où G est un substituant lié au groupe amine secondaire de spermidine, choisi parmi les groupes alkyles saturés ou insaturés, linéaires ou ramifiés constitués de 1 à 6 atomes de carbone, où un ou plusieurs atomes de carbone sont facultativement remplacés par du fluor, à savoir du méthyle, de l'éthyle, du trifluorométhyle, du trifluoroéthyle, du propyle, de l'isopropyle, du butyle, de l'isobutyle, du pentyle, de l'hexyle, de l'éthylène, du vinyle, du propylène, du butylène ; les groupes aryles ou arylalkyles, tels que le phényle, le naphtyle, le benzyle, le tolyle, où un ou plusieurs atomes de carbone sont facultativement remplacés par du fluor, et où lesdits groupes arylalkyles comprennent les groupes alkyles saturés ou insaturés, linéaires ou ramifiés constitués de 1 à 6 atomes de carbone, où un ou plusieurs atomes de carbone sont facultativement remplacés par du fluor, à savoir du méthyle, de l'éthyle, du trifluorométhyle, du trifluoroéthyle, du propyle, de l'isopropyle, du butyle, de l'isobutyle, du pentyle, de l'hexyle, de l'éthylène, du vinyle, du propylène, du butylène ; les groupes cycloalkyles saturés ou insaturés constitués par 3 à 8 atomes de carbone qui sont facultativement remplacés par des groupes alkyles saturés ou insaturés, linéaires ou ramifiés constitués de 1 à 6 atomes de carbone, où un ou plusieurs atomes de carbone sont facultativement remplacés par du fluor, à savoir du méthyle, de l'éthyle, du trifluorométhyle, du trifluoroéthyle, du propyle, de l'isopropyle, du butyle, de l'isobutyle, du pentyle, de l'hexyle, de l'éthylène, du vinyle, du propylène, du butylène.

63. Shampoing ou après-shampoing selon la revendication 62, dans lequel les groupements réactifs et les groupes thiols réagissent pour former des liaisons covalentes carbone-soufre (C-S).

64. Shampoing ou après-shampoing selon l'une quelconque des revendications 62 et 63, dans lequel A, B, C et D sont identiques.

65. Shampoing ou après-shampoing selon l'une quelconque des revendications 62 et 63, dans lequel au moins l'un de A, B, C et D est différent des autres groupements réactifs.

66. Shampoing ou après-shampoing selon l'une quelconque des revendications 62 à 65, dans lequel le lieur est une molécule polyfonctionnelle,
dans lequel le lieur est facultativement indépendamment substitué par un ou plusieurs substituants comprenant l'hydrogène, l'halogène, le cyano, l'alcoxy, l'alkyle, l'alcényle, le cycloalkyle, le cycloalcényle, l'aryle, l'hétérocycloalkyle, l'hétéroaryle, l'amine, l'hydroxy, le formyle, l'acyle, l'acide carboxylique (-COOH), -C(O)R¹, -C(O)OR¹, le carboxylate (-COO⁻), l'amide primaire (par ex., -CONH₂), l'amide secondaire (par ex.,-CONHR¹), -C(O)NR¹R², -NR¹R², -NR¹S(O)₂R², -NR¹C(O)R², -S(O)₂R², -SR¹, -S(O)₂NR¹R², le groupe sulfinyle (par ex., - SOR¹¹) et le groupe sulfonyle (par ex., -SOOR¹) ;
où R¹ et R² peuvent être chacun indépendamment de l'hydrogène, de l'alkyle, de l'alcényle, de l'alcynyle, du cycloalkyle, de l'aryle, de l'hétérocycloalkyle et de l'hétéroaryle ;
où chacun de R¹ et R² est facultativement indépendamment substitué par un ou plusieurs substituants choisis dans le groupe constitué par l'halogène, l'hydroxyle, le cyano, le nitro, l'amino, l'alkylamino, le dialkylamino, l'alkyle facultativement substitué par un ou plusieurs de l'halogène, de l'alcoxy ou de l'aryloxy, l'aryle facultativement substitué par un ou plusieurs de l'halogène, de l'alcoxy, de l'alkyle ou du trihaloalkyle, l'hétérocycloalkyle facultativement substitué par l'aryle, l'hétéroaryle ou =O, ou l'alkyle facultativement substitué par l'hydroxyle, le cycloalkyle facultativement substitué par l'hydroxyle, l'hétéroaryle facultativement substitué par un ou plusieurs de l'halogène, de l'alcoxy, de l'alkyle ou du trihaloalkyle, de l'haloalkyle, de l'hydroxyalkyle, du carboxy, de l'alcoxy, de l'aryloxy, de l'alcoxycarbonyle, de l'aminocarbonyle, de l'alkylaminocarbonyle et du dialkylaminocarbonyle.

67. Shampoing ou après-shampoing selon l'une quelconque des revendications 62 à 66, dans lequel l'agent liant est : 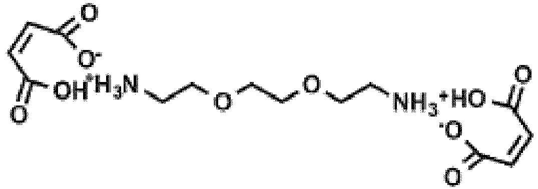 ou

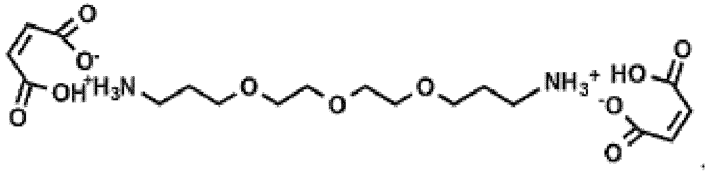

68. Shampoing ou après-shampoing selon l'une quelconque des revendications 62 à 67, comprenant en outre un ou plusieurs excipients cosmétiquement acceptables choisis dans le groupe constitué par l'eau, les tensioactifs, les vitamines, les extraits naturels, les conservateurs, les agents chélatants, les parfums, les conservateurs, les antioxydants, les agents chélatants, les colorants capillaires, les protéines, les acides aminés, les humectants, les fragrances, les émollients, les pénétrants, les épaississants, les modificateurs de viscosité, les mousses coiffantes, les agents filmogènes, les émulsifiants, les opacifiants, les propulseurs, les véhicules liquides, les vecteurs, les sels, les agents d'ajustement du pH, les agents neutralisants, les tampons, les démêlants, les agents antistatiques, les agents anti-frisottis, les agents antipelliculaires et les combinaisons de ceux-ci.

69. Shampoing ou après-shampoing selon la revendication 68, dans lequel l'agent liant est présent dans une quantité comprise entre 0,01 % en poids et 50 % en poids de la formulation.

70. Shampoing ou après-shampoing selon l'une quelconque des revendications 68 et 69, dans lequel l'agent liant est présent dans une quantité comprise entre 2,5 % et 3 % en poids de la formulation.
